# EUROPEAN PATENT SPECIFICATION

(11) **EP 1 474 121 B1**
(45) Date of publication and mention of the grant of the patent: **21.07.2010**
(21) Application number: 02734614.7
(22) Date of filing: 30.05.2002
(51) Int. Cl.: A61K 31/11, A61K 31/135, A61K 31/335

(54) **Synergistic association of (-)- gossypol with docetaxel or paclitaxel for cancer treatment**
Synergistische Kombination von (-) -Gossypol mit Docetaxel oder Paclitaxel zur Behandlung von Krebs.
Association synergique de (-)- gossypol avec du docetaxel ou du paclitaxel pour le traitement du cancer.

(30) Priority: 30.05.2001 US 293983 P
(43) Date of publication of application: 10.11.2004
(73) Proprietor: THE REGENTS OF THE UNIVERSITY OF MICHIGAN, Ann Arbor, Michigan 48109-1280 (US); GEORGETOWN UNIVERSITY, Washington, DC 20057 (US)
(72) Inventor: WANG, Shaomeng, Saline, MI 48176 (US); YANG, Dajun, Rockville, MD 20850 (US)
(74) Representative: Glawe, Delfs, Moll
(86) International application number: PCT/US2002/017206
(87) International publication number: WO 2002/097053

(56) References cited:
- US-A- 4 806 568
- US-A- 5 026 726
- US-A- 5 385 936
- US-A- 6 114 397
- DATABASE EMBASE [Online] ELSEVIER SCIENCE PUBLISHERS, AMSTERDAM, NL; 1999, BALCI A ET AL: "Gossypol induced apoptosis in the human promyelocytic leukemia cell line HL 60" XP002370267 Database accession no. EMB-1999433007 & TOHOKU JOURNAL OF EXPERIMENTAL MEDICINE 1999 JAPAN, vol. 189, no. 1, 1999, pages 51-57, ISSN: 0040-8727
- SHELLEY M D ET AL: "Stereo-specific cytotoxic effects of gossypol enantiomers and gossypolone in tumour cell lines" CANCER LETTERS, vol. 135, 1999, pages 171-180, XP002368849
- WANG X ET AL: "Cytotoxic effect of gossypol on carcinoma cells" LIFE SCIENCES, vol. 67, 2000, pages 2663-2671, XP002368850
- DATABASE BIOSIS [Online] BIOSCIENCES INFORMATION SERVICE, PHILADELPHIA, PA, US; 1989, POLSKY B ET AL: "INACTIVATION OF HUMAN IMMUNODEFICIENCY VIRUS IN-VITRO BY GOSSYPOL" XP002370268 Database accession no. PREV198988035271 & CONTRACEPTION, vol. 39, no. 6, 1989, pages 579-588, ISSN: 0010-7824
- DATABASE MEDLINE [Online] US NATIONAL LIBRARY OF MEDICINE (NLM), BETHESDA, MD, US; September 1986 (1986-09), JOSEPH A E ET AL: "Cytotoxicity of enantiomers of gossypol." XP002370269 Database accession no. NLM3756086 & BRITISH JOURNAL OF CANCER. SEP 1986, vol. 54, no. 3, September 1986 (1986-09), pages 511-513, ISSN: 0007-0920
- VAN POZNAK C ET AL: "Oral gossypol in the treatment of patients with refractory metatstatic breast cancer: A phase I/II clinical trial" BREAST CANCER RESEARCH AND TREATMENT, vol. 66, April 2001 (2001-04), pages 239-248, XP002368851
- GILBERT N E ET AL: "Antiproliferative activity of gossypol and gossypolone on human breast cancer cells" LIFE SCIENCES, vol. 57, no. 1, 1995, pages 61-67, XP002368852
- SHIDAIFAT F ET AL: "Inhibition of human prostate cancer cell growth by gossypol is assocoated with stimulation of transforming groth factor beta" CANCER LETTERS, vol. 107, 1996, pages 37-44, XP002368853
- DATABASE MEDLINE [Online] US NATIONAL LIBRARY OF MEDICINE (NLM), BETHESDA, MD, US; May 1999 (1999-05), BUSHUNOW P ET AL: "Gossypol treatment of recurrent adult malignant gliomas." XP002370270 Database accession no. NLM10448875 & JOURNAL OF NEURO-ONCOLOGY. MAY 1999, vol. 43, no. 1, May 1999 (1999-05), pages 79-86, ISSN: 0167-594X
- FLACK M R ET AL: "Oral gossypol in the treatment of metatstatic adrenal cancer" JOURNAL OF CLINICAL ENDOCRINOLOGY AND METABOLISM, vol. 76, no. 4, 1993, pages 1019-1024, XP002368854
- DATABASE EMBASE [Online] ELSEVIER SCIENCE PUBLISHERS, AMSTERDAM, NL; 2000, VANDER JAGT D L ET AL: "Gossypol: Prototype of inhibitors targeted to dinucleotide folds" XP002370271 Database accession no. EMB-2000214864 & CURRENT MEDICINAL CHEMISTRY 2000 NETHERLANDS, vol. 7, no. 4, 2000, pages 479-498, ISSN: 0929-8673
- DATABASE BIOSIS [Online] BIOSCIENCES INFORMATION SERVICE, PHILADELPHIA, PA, US; 20 October 2000 (2000-10-20), WANG XIANGHONG ET AL: "Cytotoxic effect of gossypol on colon carcinoma cells" XP002370272 Database accession no. PREV200000542709 & LIFE SCIENCES, vol. 67, no. 22, 20 October 2000 (2000-10-20), pages 2663-2671, ISSN: 0024-3205
- DAO V-T ET AL: "Synthesis and cytotoxicity of gossypol related compounds" EUROPEAN JOURNAL OF MEDICINAL CHEMISTRY, EDITIONS SCIENTIFIQUE ELSEVIER, PARIS, FR, vol. 35, no. 9, September 2000 (2000-09), pages 805-813, XP004217180 ISSN: 0223-5234
- DATABASE CAPLUS [Online] VICHKANOVA ET AL: 'Viricidal activity of gossypol in vitro', XP002999090 Retrieved from STN Database accession no. 69:94802 & ANTIBIOTICS (MOSCOW) vol. 13, no. 9, 1968, pages 828 - 829

## Description

### FIELD OF THE INVENTION

The present invention relates to naturally occurring and chemically synthesized small molecules antagonists of Bcl-2 family proteins. In particular, the present invention provides gossypol derivatives and medical uses of gossypol derivatives as antagonists of the anti-apoptotic effects of Bcl-2 and Bcl-X_{L} proteins especially in cancer cells that overexpress Bcl-2 family proteins (*e.g*., Bcl-2 and/or Bcl-X_{L}).

### BACKGROUND OF THE INVENTION

Multicellular organisms use a process called apoptosis to instruct damaged or unnecessary cells to destroy themselves for the good of the organism. Control of the apoptotic process is very important for the normal development of the organism, for example, fetal development of fingers and toes requires the controlled removal, by apoptosis, of excess interconnecting tissues, as does proper formation of neural synapses within the brain. Similarly, controlled apoptosis is responsible for the sloughing off of the inner lining of the uterus (the endometrium) at the start of menstruation.

Apoptosis not only plays an important role in tissue sculpting during development and normal cellular maintenance, it is also the primary defense against cells that pose a threat to the well being of the whole organisms. For instance, in the cell mediated immune response, effector cells (*e.g*., cytotoxic T lymphocytes "CTLs") destroy virus-infected cells by inducing the infected cells to undergo apoptosis. The organism subsequently relies on the apoptotic process to destroy the effector cells when they are no longer needed. Autoimmunity is prevented by the CTLs inducing apoptosis in each other and even in themselves. Defects in this process are associated with a variety of autoimmune diseases such as lupus erythematosus and rheumatoid arthritis.

Multicellular organisms also use the apoptotic process to instruct cells with damaged nucleic acids (*e.g.,* DNA) to destroy themselves prior to becoming cancerous. However, some cancer-causing viruses prevent apoptosis in the cells they have transformed. For example, two human papilloma viruses (HPV) have been implicated in causing cervical cancer by suppressing apoptotic removal of transformed cells by producing a protein (E6) that inactivates the p53 apoptosis promoter. Epstein-Barr virus (EBV), the causative agent of mononucleosis and Burkitt's lymphoma a solid tumor of B lymphocytes, produces a protein similar to Bcl-2 and another that causes transformed cells to increase production of Bcl-2. Both of these mechanisms make the Epstein-Barr virus transformed cells resistant to apoptosis thus allowing the cancerous cells to proliferate and to spread throughout the organism.

Some cancers that arise by non-viral means have also developed mechanisms to escape destruction by apoptosis. Melanoma cells, for instance, avoid apoptosis by inhibiting the expression of the gene encoding Apaf-1. Other cancer cells, especially lung and colon cancer cells, secrete elevated levels of soluble decoy molecules that binds to FasL, inhibiting it from binding to Fas. CTLs are thus prohibited from destroying these cancer cells. Other cancer cells express high levels of FasL, again, avoiding destruction by the CTLs. Still other viruses manipulate the cell's apoptotic machinery without directly resulting in the development of a cancer. For example, destruction of the immune system in individuals infected with the human immunodeficiency virus (HIV) is thought to progress by infected CD4⁺ T cells (about 1 in 100,000) instructing their sister cells to undergo apoptosis.

Faulty regulation of the apoptotic machinery has also been implicated in various degenerative conditions and vascular diseases.

It is apparent that the controlled regulation of the apoptotic process and the apoptotic machinery is vital to the survival of multicellular organisms. Typically, the biochemical changes that occur in a cell instructed to undergo apoptosis occur in an orderly procession. However, as shown above, flawed regulation of the apoptotic process and machinery can cause serious deleterious effects and disease to arise in an organism.

There have been various attempts to control and restore regulation of the apoptotic machinery in aberrant cells (*e.g*., cancer cells). Generally, these attempts have had limited success as treatments for the underlying diseases characterized by the faulty regulation of the apoptotic machinery for a number of reasons, such as, toxicity, ineffectiveness, high costs, and the like. What are needed are improved compositions and methods of regulating apoptosis in subjects afflicted with diseases and conditions that are characterized by faulty regulation of the apoptotic process.

### SUMMARY OF THE INVENTION

The present invention is defined in the claims and relates to naturally occurring and chemically synthesized small molecules antagonists of Bcl-2 family proteins. In particular, the present invention provides gossypol derivatives and medical uses of using gossypol derivatives as antagonists of the anti-apoptotic effects of Bcl-2 and Bcl-X_{L} proteins, especially in cancer cells that overexpress Bcl-2 family proteins (*e.g*., Bcl-2 and/or Bcl-X_{L}).

More specifically, the invention provides the use of (-) -gossypol in the manufacture of a medicament for the treatment of cancer in a subject, wherein a therapeutically effective amount of said (-)-gossypol is co-administered to said subject with an anticancer agent selected from the group consisting of docetaxel and paclitaxel.

The invention also provides (-)-gossypol for use in a method for treating cancer in a subject, wherein a therapeutically effective amount of said (-)-gossypol is co-administered to said subject with an anticancer agent selected from the group consisting of docetaxel and paclitaxel.

Bcl-2 is the founding member of a family of proteins and was first isolated as the product of an oncogene. The Bcl-2 family of proteins now includes both anti-apoptotic molecules such as Bcl-2 and Bcl-X_{L} and pro-apoptotic molecules such as Bax, Bak, Bid, and Bad. Bcl-2 and Bcl-X_{L} are thought to be important regulators of bcl-2 family mediated apoptosis.

In related embodiments, the administration of (-)-gossypol is contemplated to provide an effective treatment of neoplastic conditions and other disorders that involve the aberrant hyperproliferation of cells (*e.g.,* tumor cells).

In other related embodiments, the present invention provides medical uses or prophylaxis of cancers in a subject comprising administering to the subject (-)-gossypol in an amount effective to inhibit Bcl-2 and/or Bcl-X_{L}, thus increasing tumor suppression and/or inducing apoptosis. Preferably, (-)-gossypol is administered in conjunction with a tumor cell apoptosis promoting agent (*e.g*., Geranylgeraniol [3,7,11,15-tetramethyl-2,6,10,14-hexadecatraen-1-ol]). The present invention contemplates that increasing tumor apoptosis reestablishes normal apoptotic control associated with basal levels of Bcl-2 and/or Bcl-X_{L} expression.

The medical uses of the present invention are particularly well suited for the treatment of cancers characterized by overexpression of Bcl-2 family proteins, including, but not limited to, Bcl-2 and/or Bcl-X_{L}.

In other preferred embodiments, the medical uses of the present invention provide treatments for a number of conditions including, but not limited to, breast cancer, prostate cancer, lung cancer, lymphomas, skin cancer, pancreatic cancer, colon cancer, melanoma, ovarian cancer, brain cancer, head and neck cancer, liver cancer, bladder cancer, non-small lung cancer, cervical carcinoma, leukemia, neuroblastoma and glioblastoma, and the like.

In one preferred embodiment, the present invention provides (-)-gossypol for use in modulating apoptosis in a cell comprising: providing: a cell, wherein the cell overexpresses a Bcl-2 family protein; (-)-gossypol and treating the cell with an effective amount of (-)-gossypol under conditions such that apoptosis in the cell is modulated.

In related embodiments, the present invention provides administering (-)-gossypol to a patient having a condition characterized by overexpression of a Bcl-2 family protein. In some embodiments, the Bcl-2 family proteins contemplated include, but are not limited to, Bcl-2, Bcl-X_{L}, Mcl-1, A1/BFL-1, and BOO-DIVA, Bcl-w, Bcl-6, Bcl-8 and Bcl-y, and the like. In still some other embodiments, the Bcl-2 family proteins have pro-apoptotic activity. In yet other embodiments, the Bcl-2 family proteins have anti-apoptotic activity.

In some embodiments, diseased cells and tissues are treated with the medical uses of the present invention. In this regard, various diseases are amenable to treatment or prophylaxis with the medical uses of the invention. A non-limiting example of these diseases includes: breast cancer; prostate cancer; lung cancer; lymphomas; skin cancer; pancreatic cancer; colon cancer; melanoma; ovarian cancer; brain cancer; head and neck cancer; liver cancer; bladder cancer; non-small lung cancer; cervical carcinoma; leukemia; neuroblastoma and glioblastoma; and the like. In some embodiments, the cancer cells being treated are metastatic.

In yet another embodiment, the present invention is related to (-)-gossypol for use in treating a subject (e.g., a patient) comprising administering (-)-gossypol to a subject overexpressing a Bcl-2 family protein. In a preferred example of these embodiments, the gossypol compound binds to a Bcl-2 family protein.

Preferred embodiments of the present invention are directed at providing (-)-gossypol for use in treating a subject comprising administering (-)-gossypol and an anticancer agent to a subject overexpressing a Bcl-2 family protein.

A number of suitable anticancer agents are contemplated for use in the present invention. Indeed, the present invention contemplates, but is not limited to, administration of numerous anticancer agents such as: agents that induce apoptosis; polynucleotides (e.g., ribozymes); polypeptides (e.g., enzymes); drugs; biological mimetics; alkaloids; alkylating agents; antitumor antibiotics; antimetabolites; hormones; platinum compounds; monoclonal antibodies conjugated with anticancer drugs, toxins, and/or radionuclides; biological response modifiers (*e.g.,* interferons [*e*.*g*., IFN-α, *etc*.] and interleukins [*e*.*g*., IL-2, *etc.*], *etc*.); adoptive immunotherapy agents; hematopoietic growth factors; agents that induce tumor cell differentiation (*e.g*., all-trans-retinoic acid, etc.); gene therapy reagents; antisense therapy reagents and nucleotides; tumor vaccines; and inhibitors of angiogenesis, and the like. Numerous other examples of chemotherapeutic compounds and anticancer therapies suitable for co-administration with the disclosed gossypol compounds are known to those skilled in the art.

In preferred embodiments, anticancer agents comprise agents that induce or stimulate apoptosis. Agents that induce apoptosis include, but are not limited to, radiation (*e.g.,* UV); kinase inhibitors (*e*.*g*., Epidermal Growth Factor Receptor [EGFR] kinase inhibitor, Vascular Growth Factor Receptor [VGFR] kinase inhibitor, Fibroblast Growth Factor Receptor [FGFR] kinase inhibitor, Platelet-derived Growth Factor Receptor [PGFR] kinase inhibitor, and Bcr-Abl kinase inhibitors such as STI-571, Gleevec, and Glivec]); antisense molecules; antibodies [*e.g*., Herceptin and Rituxan]; anti-estrogens [*e.g*., raloxifene and tamoxifen]; anti-androgens [*e.g.,* flutamide, bicalutamide, finasteride, aminoglutethamide, ketoconazole, and corticosteroids]; cyclooxygenase 2 (COX-2) inhibitors [*e.g.,* Celecoxib, meloxicam, NS-398, and non-steroidal anti-inflammatory drugs (NSAIDs)]; and cancer chemotherapeutic drugs [*e.g*., irinotecan (Camptosar), CPT-11, fludarabine (Fludara), dacarbazine (DTIC), dexamethasone, mitoxantrone, Mylotarg, VP-16, cisplatinum, 5-FU, Doxrubicin, Taxotere or taxol]; cellular signaling molecules; ceramides and cytokines; and staurosprine, and the like.

In still other embodiments, the present invention is related to (-)-gossypol for use in treating cancer in a subject comprising administering to a patient having a condition characterized by overexpression of a Bcl-2 family protein an effective amount of (-)-gossypol.

Additional embodiments are directed to methods of treating cancer in a subject comprising administering to a subject having cancer, wherein the cancer is characterized by overexpression of a Bcl-2 family protein, an effective amount of (-)-gossypol and an anticancer agent.

Still other methods are directed to treating cancer in a subject comprising: administering to a patient having cancer, wherein the cancer is characterized by resistance to cancer therapies (*e.g*., chemoresistant, radiation resistant, hormone resistant, and the like), an effective amount of (-)-gossypol.

In some embodiments, the present invention is related to (-)-gossypol for use in treating cancer in a subject comprising administering to a patient having cancer, wherein the cancer is characterized by overexpression of a Bcl-2 family protein, a dose of (-)-gossypol sufficient to inhibit the activity and/or reduce the overexpression of the Bcl-2 protein.

The present invention, also provides (-)-gossypol for use in treating cancer in a subject comprising administering to a patient having cancer, wherein the cancer is characterized by overexpression of a Bcl-2 family protein, a dose of (-)-gossypol and an anticancer agent sufficient to reduce the overexpression of the Bcl-2 protein.

The present invention is also related to (-)-gossypol for use in treating cancer in a subject comprising administering to a patient having cancer, wherein said cancer is characterized by overexpression of a Bcl-2 family protein, a dose of (-)-gossypol sufficient to inhibit the activity of Bcl-2 family protein and/or reduce the overexpression of said Bcl-2 protein.

In still other embodiments, the present invention provides (-)-gossypol for use in treating cancer in a subject comprising administering to a patient having cancer, wherein said cancer is characterized by overexpression of a Bcl-2 family protein, a dose of (-)-gossypol and an anticancer agent sufficient to inhibit the activity of Bcl-2 protein and/or reduce the overexpression of said Bcl-2 protein.

Other advantages, benefits, and preferable embodiments of the present invention will be apparent to those skilled in the art.

### DESCRIPTION OF THE FIGURES

The following figures form part of the specification and are included to further demonstrate certain aspects and embodiments of the present invention. The present invention is not intended to be limited however to the embodiments specifically recited in these figures.
Fig. 1 shows a sequence alignment of Bcl-2 (SEQ ID NO:1) and Bcl-X_{L} (SEQ ID NO:2).
Fig. 2 shows a ribbon representation of the overall Bcl-2 structure in complex with the Bak BH3 peptide.
Fig. 3 shows direct inhibition of the binding between Bak BH3 peptide and Bcl-2 (Bcl-XL) by gossypol.
Fig. 4 shows the results of binding assays in one embodiment of the present invention.
Fig. 5A shows the results of binding assays in one embodiment of the present invention.
Fig. 5B shows the results of binding assays in one embodiment of the present invention.
Fig. 6 shows the results of binding assays in one embodiment of the present invention.
Fig. 7 shows the results of binding assays in one embodiment of the present invention.
Fig. 8A shows the results of binding assays in one embodiment of the present invention.
Fig. 8B shows the results of binding assays in one embodiment of the present invention.
Fig. 9 shows the results of binding assays in one embodiment of the present invention.
Fig. 10 Fig. 10 provides the chemical structures of gossypol, gossypolone, and the Schiff's bases of gossypol and gossypolone.
   results of cell based assays in one embodiment of the present invention.
Fig. 11 shows the results of binding assays in one embodiment of the present invention.
Fig. 12 shows the results of binding assays in one embodiment of the present invention.
Fig. 13 shows the results of binding assays in one embodiment of the present invention.
Fig. 14 shows the results of binding assays in one embodiment of the present invention.
Fig. 15A shows the results of binding assays in one embodiment of the present invention.
Fig. 15B shows the results of binding assays in one embodiment of the present invention.
Fig. 16 shows the results of binding assays in one embodiment of the present invention.
Fig. 17 shows the results of binding assays in one embodiment of the present invention.

### DEFINITIONS

To facilitate an understanding of the present invention, a number of terms and phrases are defined below.

As used herein, the term "gossypol compound" refers to (-)-gossypol and pharmaceutically acceptable salts of (-)-gossypol.

As used herein, the term "Bcl-2 family proteins," refers to both the anti-apoptotic members of the Bcl-2 family, including, but not limiting to Bcl-2, Bel-X_{L}, Mcl-1, A1/BFL-1, and BOO-DIVA, Bcl-w, Bcl-6, Bcl-8 and Bcl-y, and the pro-apoptotic members of the Bcl-2 family, including, but not limiting to Bak, Bax, Bad, tBid, *Harakiri*, Bim, Bmf, as well as other members of BH3 (Bcl-2 homology 3) containing proteins that are regulated by gossypol compounds.

As used herein, the terms "overexpression of Bcl-2," or "overexpression of a Bcl-2 family protein" refer to an elevated level (*e.g*., aberrant) of mRNAs encoding for a Bcl-2 family protein(s), and/or to elevated levels of Bcl-2 family protein(s) in cells or tissues as compared to similar normal corresponding nonpathological cells and tissues expressing basal levels of mRNAs encoding Bcl-2 family proteins or having basal levels of Bcl-2 family proteins. Methods for detecting the levels of mRNAs encoding Bcl-2 family proteins, or levels of Bcl-2 family proteins, in a cell or tissue, include, but are not limited to, immunohistochemical methods, and methods of nucleic acid amplification.

As used herein, the terms "anticancer agent," or "conventional anticancer agent" refer to any chemotherapeutic compounds, radiation therapies, or surgical interventions, used in the treatment of cancer.

As used herein the term, "*in vitro*" refers to an artificial environment and to processes or reactions that occur within an artificial environment. *In vitro* environments can consist of, but are not limited to, test tubes and cell cultures. The term "*in vivo*" refers to the natural environment (*e.g*., an animal or a cell) and to processes or reaction that occur within a natural environment.

As used herein, the term "host cell" refers to any eukaryotic or prokaryotic cell (*e.g.,* mammalian cells, avian cells, amphibian cells, plant cells, fish cells, and insect cells), whether located *in vitro* or *in vivo.*

As used herein, the term "cell culture" refers to any *in vitro* culture of cells. Included within this term are continuous cell lines (*e*.*g*., with an immortal phenotype), primary cell cultures, finite cell lines (*e.g*., non-transformed cells), and any other cell population maintained *in vitro,* including oocytes and embryos.

As used herein, the term "subject" refers to organisms to be treated by the methods of the present invention. Such organisms include, but are not limited to, humans. In the context of the invention, the term "subject" generally refers to an individual who will receive or who has received treatment (*e*.*g*., administration of gossypol compound(s), and optionally one or more anticancer agents) for a disease characterized by overexpression of Bcl-2 family proteins (*e.g*., Bcl-2, Bel-X_{L}, Mcl-1, A-1(Bfl-1), and Boo).

The term "diagnosed," as used herein, refers to the to recognition of a disease by its signs and symptoms (*e*.*g*., resistance to conventional cancer therapies),or genetic analysis, pathological analysis, histological analysis, and the like.

As used herein, the term "competes for binding" is used in reference to a first molecule (*e.g*., a gossypol compound) with an activity that binds to the same substrate (*e.g*., Bcl-2 and/or Bcl-X_{L}) as does a second molecule (*e.g*., a pro-apoptotic Bcl-2 family protein, such as, Bax, Bak, Bid, and Bad, *etc*.). The efficiency (*e.g*., kinetics or thermodynamics) of binding by the first molecule may be the same as, or greater than, or less than, the efficiency of the substrate binding to the second molecule. For example, the equilibrium binding constant (K_{D}) for binding to the substrate may be different for the two molecules.

As used herein, the term "antisense" is used in reference to RNA sequences that are complementary to a specific RNA sequence (*e.g*., mRNA). Included within this definition are antisense RNA ("asRNA") molecules involved in gene regulation by bacteria. Antisense RNA may be produced by any method, including synthesis by splicing the gene(s) of interest in a reverse orientation to a viral promoter that permits the synthesis of a coding strand. For example, once introduced into an embryo, this transcribed strand combines with natural mRNA produced by the embryo to form duplexes. These duplexes then block either the further transcription of the mRNA or its translation. In this manner, mutant phenotypes may be generated. The term "antisense strand" is used in reference to a nucleic acid strand that is complementary to the "sense" strand. The designation (-) (*i*.*e*., "negative") is sometimes used in reference to the antisense strand, with the designation (+) sometimes used in reference to the sense (*i*.*e*., "positive") strand. Regions of a nucleic acid sequences that are accessible to antisense molecules can be determined using available computer analysis methods.

The term "sample" as used herein is used in its broadest sense. A sample suspected of indicating a condition characterized by the overexpression of a Bcl-2 family protein may comprise a cell, tissue, or fluids, chromosomes isolated from a cell (*e.g*., a spread of metaphase chromosomes), genomic DNA (in solution or bound to a solid support such as for Southern blot analysis), RNA (in solution or bound to a solid support such as for Northern blot analysis), cDNA (in solution or bound to a solid support) and the like. A sample suspected of containing a protein may comprise a cell, a portion of a tissue, an extract containing one or more proteins and the like.

As used herein, the term "purified" or "to purify" refers, to the removal of undesired components from a sample. As used herein, the term "substantially purified" refers to molecules, either nucleic or amino acid sequences, that are removed from their natural environment, isolated or separated, and are at least 60% free, preferably 75% free, and most preferably 90% free from other components with which they are naturally associated. For example, an "isolated polynucleotide" is therefore a substantially purified polynucleotide.

As used herein, the term "genome" refers to the genetic material (*e.g*., chromosomes) of an organism or a host cell.

The term "nucleotide sequence of interest" refers to any nucleotide sequence (*e.g*., RNA or DNA), the manipulation of which may be deemed desirable for any reason (*e.g*., treat disease, confer improved qualities, *etc*.), by one of ordinary skill in the art. Such nucleotide sequences include, but are not limited to, coding sequences, or portions thereof, of structural genes (*e.g*., reporter genes, selection marker genes, oncogenes, drug resistance genes, growth factors, *etc*.), and non-coding regulatory sequences which do not encode an mRNA or protein product (*e.g*., promoter sequence, polyadenylation sequence, termination sequence, enhancer sequence, *etc*.).

"Nucleic acid sequence" and "nucleotide sequence" as used herein refer to an oligonucleotide or polynucleotide, and fragments or portions thereof, and to DNA or RNA of genomic or synthetic origin which may be single- or double-stranded, and represent the sense or antisense strand. As used herein, the terms "nucleic acid molecule encoding," "DNA sequence encoding," "DNA encoding," "RNA sequence encoding," and "RNA encoding" refer to the order or sequence of deoxyribonucleotides or ribonucleotides along a strand of deoxyribonucleic acid or ribonucleic acid. The order of these deoxyribonucleotides or ribonucleotides determines the order of amino acids along the polypeptide (protein) chain translated from the mRNA. The DNA or RNA sequence thus codes for the amino acid sequence.

The term "gene" refers to a nucleic acid (*e.g*., DNA or RNA) sequence that comprises coding sequences necessary for the production of a polypeptide or precursor (*e.g*., proinsulin). The polypeptide can be encoded by a full length coding sequence or by any portion of the coding sequence so long as the desired activity or functional properties (*e.g*., enzymatic activity, ligand binding, signal transduction, *etc*.) of the full-length or fragment are retained. The term also encompasses the coding region of a structural gene and includes sequences located adjacent to the coding region on both the 5' and 3' ends for a distance of about 1 kb or more on either end such that the gene corresponds to the length of the full-length mRNA. The sequences that are located 5' of the coding region and which are present on the mRNA are referred to as 5' untranslated sequences. The sequences that are located 3' or downstream of the coding region and which are present on the mRNA are referred to as 3' untranslated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene which are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "exogenous gene" refers to a gene that is not naturally present in a host organism or cell, or is artificially introduced into a host organism or cell.

As used herein, the term "vector" refers to any genetic element, such as a plasmid, phage, transposon, cosmid, chromosome, virus, virion, *etc*., which is capable of replication when associated with the proper control elements and which can transfer gene sequences between cells. Thus, the term includes cloning and expression vehicles, as well as viral vectors.

As used herein, the term "gene expression" refers to the process of converting genetic information encoded in a gene into RNA (*e.g*., mRNA, rRNA, tRNA, or snRNA) through "transcription" of the gene (*i.e*., via the enzymatic action of an RNA polymerase), and for protein encoding genes, into protein through "translation" of mRNA. Gene expression can be regulated at many stages in the process. "Up-regulation" or "activation" refers to regulation that increases the production of gene expression products (*i.e*., RNA or protein), while "down-regulation" or "repression" refers to regulation that decrease production. Molecules (*e.g*., transcription factors) that are involved in up-regulation or down-regulation are often called "activators" and "repressors," respectively.

As used herein, the terms "nucleic acid molecule encoding," "DNA sequence encoding," "DNA encoding," "RNA sequence encoding," and "RNA encoding" refer to the order or sequence of deoxyribonucleotides or ribonucleotides along a strand of deoxyribonucleic acid or ribonucleic acid. The order of these deoxyribonucleotides or ribonucleotides determines the order of amino acids along the polypeptide (protein) chain translated from the mRNA. The DNA or RNA sequence thus codes for the amino acid sequence.

The terms "homology" and "percent identity" when used in relation to nucleic acids refers to a degree of complementarity. There may be partial homology (*i.e*., partial identity) or complete homology (*i.e*., complete identity). A partially complementary sequence is one that at least partially inhibits a completely complementary sequence from hybridizing to a target nucleic acid sequence and is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe (*i.e.,* an oligonucleotide which is capable of hybridizing to another oligonucleotide of interest) will compete for and inhibit the binding (*i*.*e*., the hybridization) of a completely homologous sequence to a target sequence under conditions of low stringency. This is not to say that conditions of low stringency are such that non-specific binding is permitted; low stringency conditions require that the binding of two sequences to one another be a specific (*i*.*e*., selective) interaction. The absence of non-specific binding may be tested by the use of a second target which lacks even a partial degree of complementarity (*e*.*g*., less than about 30% identity); in the absence of non-specific binding the probe will not hybridize to the second non-complementary target.

As used herein the term "stringency" is used in reference to the conditions of temperature, ionic strength, and the presence of other compounds such as organic solvents, under which nucleic acid hybridizations are conducted. With "high stringency" conditions, nucleic acid base pairing will occur only between nucleic acid fragments that have a high frequency of complementary base sequences. Thus, conditions of "weak" or "low" stringency are often required with nucleic acids that are derived from organisms that are genetically diverse, as the frequency of complementary sequences is usually less. While the present invention can be practiced utilizing low stringency conditions, in a preferred embodiment, medium to high stringency conditions are employed.

"High stringency conditions" when used in reference to nucleic acid hybridization comprise conditions equivalent to binding or hybridization at 42 °C (*e.g*., overnight) in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent [50X Denhardt's contains per 500 ml, 5 g Ficoll (Type 400, Pharamcia), 5 g BSA (Fraction V; Sigma)], 100 µg/ml denatured salmon sperm DNA and 50% (V/V) of formamide. The hybridized DNA samples are then washed twice in a solution comprising 2 X SSPE, 0.1% SDS at room temperature followed by 0.1X SSPE, 1.0% SDS at 42 °C when a probe of about 500 nucleotides in length is employed. The art knows conditions that promote hybridization under conditions of high stringency (*e.g*., increasing the temperature of the hybridization and/or wash steps, the use of formamide in the hybridization solution, *etc*.).

"Medium stringency conditions" when used in reference to nucleic acid hybridization comprise conditions equivalent to binding or hybridization at 42 °C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent [50X Denhardt's contains per 500 ml, 5 g Ficoll (Type 400, Pharamcia), 5 g BSA (Fraction V; Sigma)] 100 µg/ml denatured salmon sperm DNA, and 50% (V/V) formamide. followed by washing in a solution comprising 1.0X SSPE, 1.0 % SDS at room temperature followed by 2X SSPE, 0.1 % SDS at 42 °C when a probe of about 500 nucleotides in length is employed.

"Low stringency conditions" comprise conditions equivalent to binding or hybridization at 42 °C in a solution consisting of 5X SSPE (43.8 g/l NaCl, 6.9 g/l NaH₂PO₄ H₂O and 1.85 g/l EDTA, pH adjusted to 7.4 with NaOH), 0.1% SDS, 5X Denhardt's reagent [50X Denhardt's contains per 500 ml, 5 g Ficoll (Type 400, Pharamcia), 5 g BSA (Fraction V; Sigma)], 100 g/ml denatured salmon sperm DNA, and 50%(V/V) formamide. followed by washing in a solution comprising 5X SSPE, 0.1 % SDS at 42 °C when a probe of about 500 nucleotides in length is employed. In addition, the art knows well that numerous equivalent conditions may be employed to comprise low stringency conditions; factors such as the length and nature (DNA, RNA, base composition) of the probe and nature of the target (DNA, RNA, base composition, present in solution or immobilized, *etc*.) and the concentration of the salts and other components (*e.g*., the presence or absence of formamide, dextran sulfate, polyethylene glycol) are considered and the hybridization solution may be varied to generate conditions of low stringency hybridization different from, but equivalent to, the above listed conditions.

When used in reference to a single-stranded nucleic acid sequence, the term "substantially homologous" refers to any probe that can hybridize (i.e., it is the complement of) the single-stranded nucleic acid sequence under conditions of low stringency as described above. A gene may produce multiple RNA species that are generated by differential splicing of the primary RNA transcript. cDNAs that are splice variants of the same gene will contain regions of sequence identity or complete homology (representing the presence of the same exon or portion of the same exon on both cDNAs) and regions of complete non-identity (for example, representing the presence of exon "A" on cDNA 1 wherein cDNA 2 contains exon "B" instead). Because the two cDNAs contain regions of sequence identity they will both hybridize to a probe derived from the entire gene or portions of the gene containing sequences found on both cDNAs; the two splice variants are therefore substantially homologous to such a probe and to each other. The present invention is not limited to the situation where hybridization takes place only between completely homologous sequences. In some embodiments, hybridization takes place with substantially homologous sequences.

As used herein, the term "protein of interest" refers to a protein encoded by a nucleic acid of interest.

As used herein, the term "native" (or wild type) when used in reference to a protein, refers to proteins encoded by partially homologous nucleic acids so that the amino acid sequence of the proteins varies. As used herein, the term "variant" encompasses proteins encoded by homologous genes having both conservative and nonconservative amino acid substitutions that do not result in a change in protein function, as well as proteins encoded by homologous genes having amino acid substitutions that cause decreased (*e.g*., null mutations) protein function or increased protein function.

The term "reverse Northern blot" as used herein refers to the analysis of DNA by electrophoresis of DNA on agarose gels to fractionate the DNA on the basis of size followed by transfer of the fractionated DNA from the gel to a solid support, such as nitrocellulose or a nylon membrane. The immobilized DNA is then probed with a labeled oligo-ribonucleotide probe or RNA probe to detect DNA species complementary to the ribo probe used.

As used herein, the term "pathogen" refers a biological agent that causes a disease state (*e.g*., infection, cancer, *etc*.) in a host. "Pathogen" include, but are not limited to, viruses, bacteria, archaea, fungi, protozoans, mycoplasma, and parasitic organisms.

As used herein, the term "organism" is used to refer to any species or type of microorganism, including but not limited to, bacteria, archaea, fungi, protozoans, mycoplasma, and parasitic organisms. As used herein, the term "fungi" is used in reference to eukaryotic organisms such as the molds and yeasts, including dimorphic fungi.

As used herein, the term "virus" refers to minute infectious agents, which with certain exceptions, are not observable by light microscopy, lack independent metabolism, and are able to replicate only within a living host cell. The individual particles (i.e., virions) consist of nucleic acid and a protein shell or coat; some virions also have a lipid containing membrane. The term "virus" encompasses all types of viruses, including animal, plant, phage, and other viruses.

The terms "bacteria" and "bacterium" refer to all prokaryotic organisms, including those within all of the phyla in the Kingdom Procaryotae. It is intended that the term encompass all microorganisms considered to be bacteria including Mycoplasma, Chlamydia, Actinomyces, Streptomyces, and Rickettsia. All forms of bacteria are included within this definition including cocci, bacilli, spirochetes, spheroplasts, protoplasts, *etc*. Also included within this term are prokaryotic organisms which are gram negative or gram positive. "Gram negative" and "gram positive" refer to staining patterns with the Gram-staining process which is well known in the art. (*See e.g*., Finegold and Martin, Diagnostic Microbiology, 6th Ed., CV Mosby St. Louis, pp 13-15 [1982]). "Gram positive bacteria" are bacteria which retain the primary dye used in the Gram stain, causing the stained cells to appear dark blue to purple under the microscope. "Gram negative bacteria" do not retain the primary dye used in the Gram stain, but are stained by the counterstain. Thus, gram negative bacteria appear red.

As used herein, the term "antigen binding protein" refers to proteins which bind to a specific antigen. "Antigen binding proteins" include, but are not limited to, immunoglobulins, including polyclonal, monoclonal, chimeric, single chain, and humanized antibodies, Fab fragments, F(ab')2 fragments, and Fab expression libraries. Various procedures known in the art are used for the production of polyclonal antibodies. For the production of antibody, various host animals can be immunized by injection with the peptide corresponding to the desired epitope including but not limited to rabbits, mice, rats, sheep, goats, *etc.* In a preferred embodiment, the peptide is conjugated to an immunogenic carrier (*e.g*., diphtheria toxoid, bovine serum albumin (BSA), or keyhole limpet hemocyanin [KLH]). Various adjuvants are used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and Corynebacterium parvum.

For preparation of monoclonal antibodies, any technique that provides for the production of antibody molecules by continuous cell lines in culture may be used (*See e.g.,* Harlow and Lane, Antibodies: A Laboratory Manual, Cold Spring Harbor Laboratory Press, Cold Spring Harbor, NY). These include, but are not limited to, the hybridoma technique originally developed by Köhler and Milstein (Köhler and Milstein, Nature, 256:495-497 [1975]), as well as the trioma technique, the human B-cell hybridoma technique (*See e.g*., Kozbor et al., Immunol. Today, 4:72 [1983]), and the EBV-hybridoma technique to produce human monoclonal antibodies (Cole et al., in Monoclonal Antibodies and Cancer Therapy, Alan R. Liss, Inc., pp. 77-96 [1985]).

According to the invention, techniques described for the production of single chain antibodies (U.S. 4,946,778) can be adapted to produce specific single chain antibodies as desired. An additional embodiment of the invention utilizes the techniques known in the art for the construction of Fab expression libraries (Huse et al., Science, 246:1275-1281 [1989]) to allow rapid and easy identification of monoclonal Fab fragments with the desired specificity.

Antibody fragments that contain the idiotype (antigen binding region) of the antibody molecule can be generated by known techniques. For example, such fragments include but are not limited to: the F(ab')2 fragment that can be produced by pepsin digestion of an antibody molecule; the Fab' fragments that can be generated by reducing the disulfide bridges of an F(ab')2 fragment, and the Fab fragments that can be generated by treating an antibody molecule with papain and a reducing agent.

Genes encoding antigen binding proteins can be isolated by methods known in the art. In the production of antibodies, screening for the desired antibody can be accomplished by techniques known in the art (*e*.*g*., radioimmunoassay, ELISA (enzyme-linked immunosorbant assay), "sandwich" immunoassays, immunoradiometric assays, gel diffusion precipitin reactions, immunodiffusion assays, *in situ* immunoassays (using colloidal gold, enzyme or radioisotope labels, for example), Western Blots, precipitation reactions, agglutination assays (*e.g*., gel agglutination assays, hemagglutination assays, *etc*.), complement fixation assays, immunofluorescence assays, protein A assays, and immunoelectrophoresis assays, *etc*.) *etc.*

As used herein, the term "instructions for administering said gossypol compound to a subject" includes instructions for using the compositions contained in the kit for the treatment of conditions characterized by the overexpression of a Bcl-2 family protein in a cell or tissue. The term also refers to instructions for using the compositions contained in the kit to treat cancers characterized as being resistant to at least one conventional anticancer therapy (*e.g*., chemotherapy). In some embodiments, the instructions further comprise a statement of the recommended or usual dosages of the compositions contained within the kit pursuant to 21 CFR §201 *et seq.* Additional information concerning labeling and instruction requirements applicable to the methods and compositions of the present are available at the Internet web page of the U.S. FDA.

The term "test compound" refers to any chemical entity, pharmaceutical, drug, and the like, that can be used to treat or prevent a disease, illness, sickness, or disorder of bodily function, or otherwise alter the physiological or cellular status of a sample (e.g., the level of Bcl-2 family proteins in a cell). Test compounds comprise both known and potential therapeutic compounds. A test compound can be determined to be therapeutic by using the screening methods of the present invention. A "known therapeutic compound" refers to a therapeutic compound that has been shown (*e.g*., through animal trials or prior experience with administration to humans) to be effective in such treatment or prevention. In preferred embodiments, "test compounds" are anticancer agents. In particularly, preferred embodiments, "test compounds" are anticancer agents that induce apoptosis in cells.

As used herein, the term "third party" refers to any entity engaged in selling, warehousing, distributing, or offering for sale a test compound contemplated for co-administered with a gossypol compound for treating conditions characterized by the overexpression of the Bcl-2 family proteins.

As used herein, the term "modulate" refers to the activity of a compound (*e.g*., gossypol compound) to affect (*e.g*., to promote or retard) an aspect of the cellular function, including, but not limited to, cell growth, proliferation, apoptosis, and the like.

### GENERAL DESCRIPTION OF THE INVENTION

Gossypol is a naturally occurring double biphenolic compound derived from crude cotton seed oil (*Gossypium sp*.). Human trials of gossypol as a male contraceptive have demonstrated the safety of long term administration of these compounds. Gossypol has been shown to be well tolerated in humans and has a low, but measurable, response rate in heavily pretreated poor-prognosis patients with recurrent glioma.

The present invention is defined in the claims and provides *in vivo* data that show gossypol compounds significantly inhibit tumor growth, but that in some embodiments gossypol compounds achieve even greater inhibition of tumor growth when used in combination (co-administration) with one or more conventional anticancer agents (*e.g*., docetaxel). Accordingly, in preferred embodiments, gossypol compounds, and metabolites, enantiomers, and derivatives thereof, are administered to patients suffering from diseases characterized by overexpression of Bcl-2 and/or Bcl-X_{L} (*e.g*., cancer). Gossypol induces apoptosis in cancer cells expressing high levels of Bcl-2 and/or Bcl-XL, but gossypol has little affect on cells with low levels Bcl-X_{L} and/or Bcl-2 expression.

Gossypol is a known inhibitor of spermatogenesis that may be administered orally with few side effects. Some researchers have shown, however, that hypoklemia may result from prolonged gossypol administration. Accordingly, in some embodiments, the present methods and compositions further comprise the co-administered of potassium supplements to patients being treated with gossypol compounds.

Gossypol has been studied as an anticancer therapeutic since the 1980s in *in vitro* and *in vivo* models. Prior to the present invention, however, the mechanisms of gossypol's anticancer effects were not known. The present discovery that gossypol is a potent inhibitor for Bcl-2 and Bcl-X_{L} shows that the anti-tumor activity of gossypol is due, at least in part, to inhibition of the anti-apoptotic activity of Bcl-2 and Bcl-X_{L} and the subsequent induction of apoptosis in cancer cells expressing high levels of Bcl-2 family proteins. Thus, the present invention provides compositions and methods for targeting subjects characterized as overexpressing a Bcl-2 family protein.

In the clinical trials to date, gossypol has shown low toxicity in patients. In some embodiments, it is contemplated that gossypol compounds provide efficient single agent treatment for metastatic cancers. The present invention further contemplates that gossypol compounds represent new classes of anticancer agents that specifically antagonize the anti-apoptotic effects of Bcl-2 and Bcl-X_{L}.

Bcl-2 is the founding member of a family of proteins that includes both anti-apoptotic molecules (*e.g*., Bcl-2, Bcl-X_{L}, Mcl-1, A1/BFL-1, and BOO-DIVA, Bcl-w Bcl-6, Bcl-8 and Bcl-y and the like) and pro-apoptotic molecules (*e.g*., Bax, Bak, Bid, and Bad, and the like). The *bcl-2* gene is a human proto-oncogene located on chromosome 18. The *bcl*-2 gene was discovered as a translocated locus in a B-cell leukemia. This translocation is also found in some B-cell lymphomas. In cancerous B cells, the portion of chromosome 18 containing the *bcl*-2 locus undergoes a reciprocal translocation with the portion of chromosome 14 containing the antibody heavy chains. This t(14;18) translocation places the *bcl*-2 gene close to the heavy chain gene enhancer. The product of the *bcl*-2 gene, Bcl-2 protein, is an integral membrane protein found in the membranes of the endoplasmic reticulum (ER), nuclear envelope, and the outer membrane of mitochondria. It is contemplated that Bcl-2, and Bcl-X_{L}, function as crucial antagonists of apoptosis.

Although an understanding of the mechanism is not necessary to practice the present invention and the present invention is not so limited, it is contemplated that anti-apoptotic proteins Bcl-2 and Bcl-X_{L} suppress apoptosis by forming heterodimers with pro-apoptotic Bcl-2 family members such as Bak, Bad, Bax, Mtd (Bok), Bim, Hrk (DP5), Blk, Bnip3, Bnip3L, and Diva. Additional anti-apoptotic members (or related proteins) of the Bcl-2 family are thought to include, but are not limited to, Mcl-1, A1/BFL-1, and BOO-DIVA, Bcl-w, Bcl-6, Bcl-8 and Bcl-y.

In some embodiments, the present invention is related to BH3 domain-containing proteins as targets for inhibition. It should be understood that where the specification refers to Bcl-2 families of proteins, the same disclosure pertains to BH3 domain-containing proteins. Thus, in some embodiments, the present invention is related to compositions and methods for the regulation of biological conditions related to the aberrant expression of BH3 domain-containing proteins.

Bcl-2 and Bcl-X_{L} are highly homologous proteins. Many forms of human cancers (*e.g*., myeloid leukemia and breast cancer) overexpress Bcl-2, and/or Bcl-X_{L}. Both Bcl-2 and Bcl-X_{L} have been found to be overexpressed in human breast cancers. In particular, Bcl-2 is found to be overexpressed in 60-80% of human breast cancers. The expression of Bcl-2 is highly correlated with estrogen receptor (ER) positive breast cancer. Bcl-X_{L} is overexpressed in 40-70% of human breast cancers, 30-60% of prostate cancers, 80% of B-cell lymphomas, 90% of colorectal adenocarcinomas, and many other forms of cancer. Bcl-X_{L} is mainly found in ER negative breast cancer. The expression of Bcl-X_{L} is typically correlated with a poor prognosis and shortened survival.

Several lines of evidence indicate that Bcl-2 and Bcl-X_{L} not only contribute to cancer progression, but also may confer cancer-resistance to apoptosis induced by conventional anti-cancer therapies. High levels of intracellular Bcl-2 protect cells (*e.g*., cancer cells) from being destroyed by the apoptosis. The majority of solid tumors are protected by at least one of the anti-apoptotic Bcl-2 proteins. Most of the currently available cancer chemotherapeutic agents target cellular DNA integrity or replication, and indirectly trigger apoptosis in tumor cells. Cancers that express high levels of Bcl-2 and/or Bcl-X_{L}, are often resistant to chemotherapeutic agents or radiation therapy.

However, the expression patterns of Bcl-2 and Bcl-X_{L} are different in some of the cancers that overexpress Bcl-2 family proteins. Several reports suggest that expression of either Bcl-2 or Bcl-X_{L} proteins is sufficient for cancer cells to show Bcl-2 family mediated resistance to chemotherapy or radiation therapy. (*See*, J.C. Reed, Pharmacology, 41:501-553 [1997]; J.C. Reed et al., J. Cell Biochem., 6:23-32 [1996[). Additional research suggests that some cancer cells are able to switch from overexpression of Bcl-2 to Bcl-X_{L}. *(See,* Z. Han et al., Cancer Res., 56:621-628 [1996]). Accordingly, some embodiments of the present invention are related to administering a therapeutic amount of one or more Bcl-2 antagonists (*e.g*., small molecules) to patients having a cancer characterized by overexpression of Bcl-2. Similarly, other embodiments of the present invention are related to administering a therapeutic amount of one or more Bcl-X_{L} antagonists (*e.g*., small molecules) to patients having a cancer characterized by overexpression of Bcl-X_{L}. In still further embodiments, the present invention is related to administering a combination of two or more Bcl-2 family antagonists (*e.g*., small molecules) to a patient having a cancer characterized by the overexpression of Bcl-2 family proteins. The present invention further contemplates providing compositions and methods comprising one or more antagonists to Bcl-2 family protein(s) (*e.g*., an anti-apoptotic Bcl-2 family protein) and one or more additional anticancer agents (*e.g.,* taxol, docetaxel, *etc*.).

Research into the three-dimensional (3D) structures of Bcl-2 and Bcl-X_{L} showed that both molecules have a hydrophobic binding pocket (called the BH3 binding pocket), that is important to their anti-apoptotic affects. Further research shows that Bak, Bad and Bax have binding domain (named the BH3 binding domain) that allows the molecule to bind to the BH3 pockets in Bcl-2 and Bcl-X_{L}. In particular, experimental 3D high resolution structures of Bcl-X_{L} (S.W. Muchmore et al., Nature, 381:335-341 [1996]; and M. Aritomi et al., J. Biol. Chem., 272:27886-27892 [1997]) alone and in complex with a Bak BH3 (Bcl-2 homology domain 3) peptide (S. Michael et al., Science, 275:983-986 [1997]) have been determined. Bcl-2 and Bcl-X_{L} share a high degree of homology in their amino acid sequences (45% of identity and 56% of similarity). It has been demonstrated that when there exists a sequence identity more than 30% between the target protein (Bcl-2) and the template protein (Bcl-XL), current computational homology modeling methods, such as MODELLER (A. Sali et al., Structure, Function, and Genetics, 23:318-326 [1995]) provide an accurate 3D structure for the target protein. *(See,* A. Sali, Curr. Opin. Biotech., 6:437-451 [1995]). Therefore, in preferred embodiments of the present invention, computational homology modeling is used to model the 3D structure of Bcl-2 (the target protein) based upon the experimental 3D structural coordinates of Bcl- XL (the template protein).

High resolution 3D NMR structural analysis of gossypol complexed with Bcl-X_{L} reveals that gossypol also binds to Bcl-X_{L} via the BH3 binding pocket. Empirical structure-based testing methods revealed that gossypol, and enantiomers thereof, are potent inhibitors of the anti-apoptotic affects of Bcl-X_{L} and to a lesser extent, the anti-apoptotic affects of Bcl-2.

Thus, inhibition of the anti-apoptotic function of Bcl-2/ Bcl-X_{L} represents a novel and promising strategy for overcoming the resistance of some cancers to chemotherapy or radiation therapy.

### DETAILED DESCRIPTION OF THE INVENTION

The present invention is defined in the claims and relates to naturally occurring and chemically synthesized small molecules antagonists of Bcl-2 family proteins. In particular, the present invention is related to (-)-gossypol as an antagonist of the anti-apoptotic effects of Bcl-2 and Bcl-X_{L} proteins especially in cancer cells that overexpress Bcl-2 family proteins (*e.g*., Bcl-2 and/or Bcl-X_{L}). Exemplary compositions and medical uses of the present invention are described in more detail in the following sections: I. Binding activity of Bcl-2 and Bcl-X_{L}; II. Structure-based approach for discovery of small molecule inhibitors of Bcl-2 and Bcl-X_{L}; III. Characterization of Bcl-2 family of proteins in cancer cell lines; IV. Gossypol inhibits cancer cell growth and proliferation; V. Proposed mechanism of gossypol activity; VI. Activity of gossypol in MDA-231 xenograft mice alone and in combination with conventional anticancer agents, VII. Therapeutic Agents combined or co-administered with gossypol; and VII. Pharmaceutical formulations, administration routes, and dosing considerations.

More specifically, the invention provides the use of (-)-gossypol in the manufacture of a medicament for the treatment of cancer in a subject, wherein a therapeutically effective amount of said (-)-gossypol is co-administered to said subject with an anticancer agent selected from the group consisting of docetaxel and paclitaxel.

The invention also provides (-)-gossypol for use in a method for treating cancer in a subject, wherein a therapeutically effective amount of said (-)-gossypol is co-administered to said subject with an anticancer agent selected from the group consisting of docetaxel and paclitaxel.

### I. Binding activity of Bcl-2 and Bcl-X_{L}

Although an understanding of the mechanism is not necessary to practice the present invention and the present invention is not so limited, it is contemplated that the anti-apoptotic affects of Bcl-2 and Bcl-X_{L} proteins is attributed, at least in part, to their ability to heterodimerize with pro-apoptotic Bcl-2 family members such as Bak, Bax and Bad. The experimental structures of Bcl-2 and Bcl-X_{L} showed that BH1 (Bcl-2 homology domain 1), BH2 and BH3 domains of Bcl-2 and Bcl-XL form a hydrophobic binding pocket (the BH3 binding pocket) into which Bak or Bad BH3 domain binds. (*See e.g*., S.W. Muchmore et al., Nature, 381:335-341 [1996]; M. Aritomi et al., J. Biol. Chem., 272:27886-27892 [1997]; S. Michael et al., Science, 275:983-986 [1997]; and A.M. Petros et al., Protein Sci., 9:2528-2534 [2000]; and A.M. Petros et al., Proc. Natl. Acad. Sci. USA, 98:3012-3017 [2001]). This binding pocket in Bcl-2/Bcl-X_{L} is essential for anti-apoptotic function. (*See e.g*., X.M. Yin et al., Nature, 369:321-323 [1994]; S.C. Cosulich et al., Curr. Biol., 7:913-920 [1997]; S. Michael *et al*., *supra*; and A.M. Petros *et al., supra*). Thus, the present invention contemplates that small molecules that bind to the BH3 binding site in Bcl-2 and/or Bcl-X_{L} are capable of blocking the hetero-dimerization of Bcl-2 and/or Bcl-X_{L} with the pro-apoptotic members of the Bcl-2 protein family (*e.g*., Bad, Bak, and Bax *etc*.) such that the anti-apoptotic function of Bcl-2 and/or Bcl-X_{L} is antagonized and apoptosis is induced in cells with Bcl-2 and/or Bcl-X_{L} overexpression. In preferred embodiments, the present invention provides small molecule antagonists that bind to the binding pocket(s) (*e.g*., BH3) of Bcl-2 and/or Bcl-X_{L}. In some of these embodiments, the present invention further provides one or more additional anticancer agents (*e.g*., taxol, docetaxel, and the like) administered in combination with the disclosed small molecules inhibitors (*e.g*., gossypol compounds) of Bcl-2 and/or Bcl-X_{L}. In particularly preferred embodiments, gossypol compounds are administered in combination with at least one anticancer agent that induces apoptosis.

The present invention contemplates that small molecule inhibitors of Bcl-2 and Bcl-X_{L} have several advantages over other commonly available protein antagonists such as antisense oligonucleotides, antibodies, and peptides, including, but not limited to, better oral availability, better stability and lower cost.

### II. Structure-based approach for discovery of small molecule inhibitors of Bcl-2 and Bcl-X_{L}

The present invention used a powerful structure-based virtual screening methodology to identify small molecule antagonists of anti-apoptotic Bcl-2 family proteins, such as Bcl-2 and Bcl-X_{L}, from large 3D chemical databases. This approach uses computational docking methods to identify potential small organic molecule inhibitors that bind to binding sites in the target proteins (*e.g*., BH3 in Bcl-2 and Bcl-X_{L}). In one embodiment, Bcl-X_{L} protein was treated using the united-atom approximation in the docking studies. Only polar hydrogens were added to the protein, and Kollman united-atom partial charges were assigned. All water molecules were removed. Atomic solvation parameters and fragmental volumes were assigned to the protein atoms using the AutoDock utility, AddSol (*See,* AutoDock Web page; G. Morris et al., J. Computational Chemistry, 19:1639-1662 [1998]).

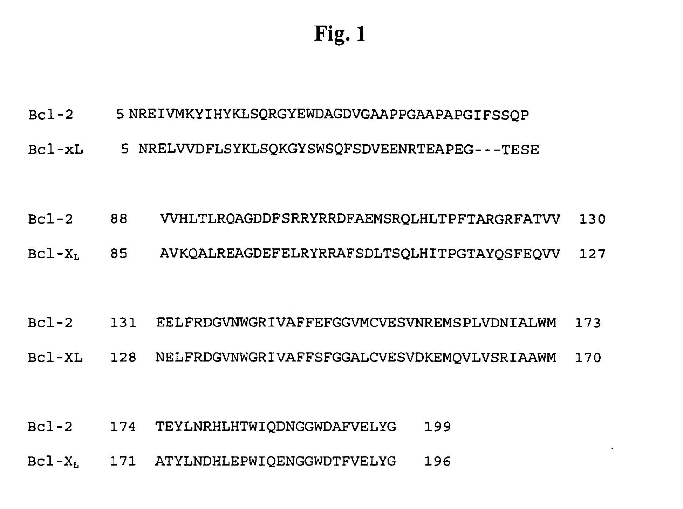

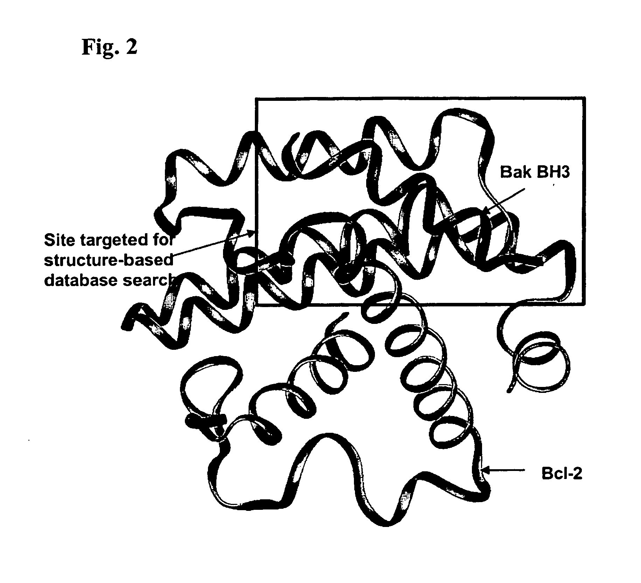

In another embodiment, the 3D structure of Bcl-2 was modeled the MODELLER homology modeling method. (A. Sali et al., Structure, Function, and Genetics, 23:318-326 [1995]; and A. Sali, Curr. Opin. Biotech., 6:437-451 [1995]). The sequence alignment obtained using the BLAST program between Bcl-2 and Bcl-X_{L} is provided in Fig. 1 and was used in the homology modeling in the present invention. Since the Bak BH3 peptide binds to both Bcl-2 and Bcl-X_{L} with good affinities (*See*, J.L. Wang et al., Cancer Res., 60:1498-1502 [2000]; and J.L. Wang et al., Proc. Natl. Acad. Sci. USA, 97:7124-7129 [2000]), the 3D structure of Bcl-2 in complex with the Bak BH3 peptide was modeled based upon the experimental NMR structure of Bcl-X_{L} complexed with Bak BH3 peptide. (S. Michael et al., Science, 275:983-986 [1997]). The modeled 3D complex structure was further refined through molecular dynamics (MD) simulation in explicit water using the CHARMM program (B.R. Brooks et al., J. Comp. Chem., 4,187-217 [1983]; and P.V.R. Schleyer et al., CHARMM: The Energy Function and Its Parameterization with an Overview of the Program, in The Encyclopedia of Computational Chemistry, 1:271-277 eds., John Wiley & Sons, Chichester [1998]) with the MSI CHARMM force field. The refined structure of Bcl-2 in complex with the Bak BH3 peptide is depicted in Fig 2. Fig. 2 shows a ribbon representation of the overall Bcl-2 structure in complex with the Bak BH3 peptide. Potential inhibitors are confirmed through biochemical and biological assays.

As compared to random screening, structure-based 3D-database searching is very effective and low-cost. A three-dimensional database containing approximately 7,000 small organic compounds that were identified and isolated from Herbal medicines was built and used in the current structure-based database screening using the program DOCK (S. Makino and I.D. Kuntz, J. Comput. Chem. 18:1812-1825 [1997]). The top 250 compounds that have the highest DOCK score were considered as potential Bcl-2 and Bcl-XL inhibitors. Of the molecules initially selected, 9 compounds were available from commercial sources and were obtained for further in vitro binding assays.

Further testing of the potential non-peptide small molecule inhibitors was carried using an established sensitive and quantitative *in vitro* fluorescence polarization (FP) based binding assay. *(See,* I.J. Enyedy et al., J. Med. Chem., 44: 313-4324 [2001]). The 9 candidate compounds were screened using this methodology for their ability to compete with the Bak BH3 peptide in binding to Bcl-2. From the 9 compounds, three compounds were found to display IC₅₀ values better than 25 µM; The IC₅₀ value of the natural Bak BH3 peptide was found to be 0.3 µM in the FP binding assays. Each of these three small molecules identified was shown to block the binding of Bcl-2 and Bak BH3 peptide. Gossypol was found to have a binding affinity of 10 M to Bcl-2 and compete with the Bak peptide.

Since Bcl-X_{L} and Bcl-2 have similar 3D structures, it was reasoned that some of the potential Bcl-2 inhibitors would also bind to Bcl-X_{L}. Accordingly, additional screening efforts were directed at discovering potential non-peptide small molecule antagonists of Bcl-X_{L} using the FP based binding assay described above.

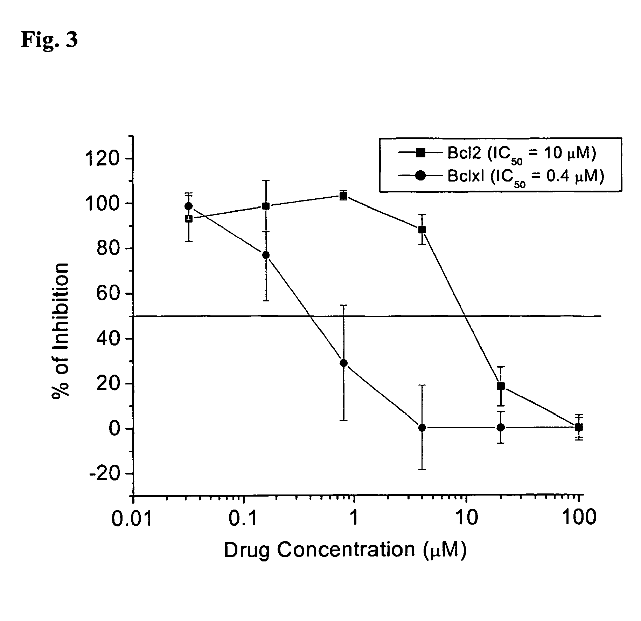

Although Bcl-2 and Bcl-X_{L} both have anti-apoptotic functions , these two proteins have different expression patterns in human cancers. Furthermore, although Bcl-2 and Bcl-X_{L} share structurally similar BH3 binding sites, there are some differences. It was discovered that while some small molecule inhibitors of Bcl-2 also have good binding affinity for Bcl-X_{L}, some other small molecule inhibitors only weakly bind Bcl-X_{L} (although a weak binder may still find use as an inhibitor). Reciprocally, some relative weak Bcl-2 small molecule inhibitors have much higher potency binding to Bcl-X_{L}. In particular, it was found that gossypol, a moderately potent inhibitor of Bcl-2, displayed a potent activity binding to Bcl-X_{L} with an IC₅₀ value of 0.4 µM (Fig. 3). Fig. 3 shows direct inhibition of the binding between Bak BH3 peptide and Bcl-2 (Bcl-XL) by gossypol as measured by FP based binding assay. The non-FP labeled Bak peptide has an IC₅₀ value of 0.3 µM to Bcl-X_{L}. In the present FP based Bcl-X_{L} binding assays, it was determined that Bak peptide has an IC₅₀ value 0.3 µM binding to Bcl-X_{L}. Thus, gossypol is a potent inhibitor for Bcl-X_{L}, having a potency similar to that of Bak peptide and it is also a moderately potent inhibitor for Bcl-2.

### III. Characterization of Bcl-2 family of proteins in cancer cell lines

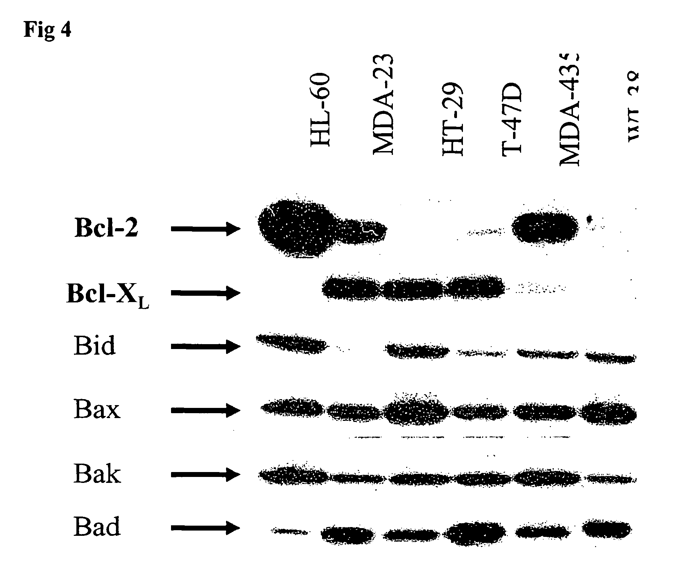

To better understand the molecular mechanism of small molecule inhibitors of Bcl-2 and Bcl-X_{L}, the expression of Bcl-2 family proteins was characterized in various breast cancer cell lines, and other cancer cell lines, from the National Cancer Institute's anti-cancer drug screening program. The results from 5 representative cancer cell lines (*i.e.,* the MDA-MB-231, T-47D, and MDA-435, breast cancer cell lines, the HL-60 leukemia cell line, and the HT-29 colon cancer cell line) and 1 normal fibroblast (WI-38) cell lines are shown in Fig. 4. The HL-60 leukemia cell line had the highest level of Bcl-2 expression, while the MDA-MB-231 and MDA-MB-435 breast cancer cell lines also showed very high levels of Bcl-2. Breast cancer cell lines MDA-MB-231 and T-47D, as well as colon cancer cell line HT-29 also showed very high levels of Bcl-X_{L} expression. The normal fibroblast cell line showed low level of Bcl-2 and Bcl-X_{L}.

The Bcl-2 family of proteins acts as arbiters of programmed cell death. The balance between anti-apoptotic molecules (*e.g*., Bcl-2 and Bcl-X_{L}) and pro-apoptotic molecules (*e.g*., Bid, Bax, Bak and Bad) plays an important role in apoptosis. For this reason, the expression status of pro-apoptotic proteins Bid, Bax, Bak and Bad in cancer cell lines MDA-MB-231, T-47D, MDA-435, HL-60, and HT-29, and normal fibroblast cell line WI-38 were also determined. (Fig. 4). All of the cell lines tested, including the normal fibroblast cell line WI-38, express high levels of Bax and most of the cancer cell lines also express high levels of Bak. There is significant variations in the expression levels of Bid and Bad between different cell lines. Taken together, all 5 cancer cell lines tested showed high levels of both anti-apoptotic and pro-apoptotic Bcl-2 family proteins, while the normal fibroblast cell line (WI-38) showed low levels of Bcl-2 and Bcl-X_{L}, but high levels of pro-apoptotic Bcl-2 family proteins Bax and Bad.

Several other breast cancer cell lines used by the National Cancer Institute (NCI) in their anticancer drug screening efforts were also tested. These cell lines include BT-549, HS 578T, MCF-7 and NCI/ADR-Resistant. Of these, MCF-7 and BT-549 have high levels of Bcl-2 protein, while BT-549 also has a high level of Bcl-X_{L} protein. HS 578T and NCI/ADR-RES have medium levels of Bcl-XL protein. Thus, among the 7 human breast cancer cell lines examined (*i.e*., MDA-MB-231, T-47D, MDA-435, BT-549, HS 578T, MCF-7, and NCI/ADR-Resistant) 5 of the cell lines have high levels of either, or both, Bcl-2 and Bcl-X_{L} expression. Two breast cancer cell lines have medium levels of Bcl-2 or Bcl-X_{L} expression. None of the 7 breast cancer cell lines tested had low Bcl-2 and low Bcl-X_{L} expression.

### IV. Gossypol inhibits cancer cell growth and proliferation

Fluorescence polarization assays showed that gossypol displaces the binding of Bak BH3 peptide to Bcl-2 and to Bcl-X_{L}. Thus, the present invention contemplates that a small molecule inhibitor (*e.g*., gossypol) that binds to the BH3 binding domain in Bcl-2 or in Bcl-X_{L} blocks the anti-apoptotic functions of these molecules which in turn induces apoptosis in cancer cells with elevated Bcl-2 and/or Bel-X_{L} expression. It is further contemplated that small molecule inhibitors (*e.g*., gossypol) will also decrease viability and proliferation in cancer cells with high Bcl-2 and/or Bcl-X_{L} expression. Gossypol inhibits cell proliferation (growth) in cancer, and more particularly, in a human breast cancers (*e.g.*, MDA-MB-231). The MDA-MB-231 breast cancer cell line has a high level of both Bcl-2 and Bcl-X_{L} expression. The ability of gossypol to inhibit MDA-MB-231 cell growth was tested in a 5 day MTT assay. Gossypol was shown to inhibit MDA-MB-231 cell growth with an IC₅₀ value of 2.0 µM.

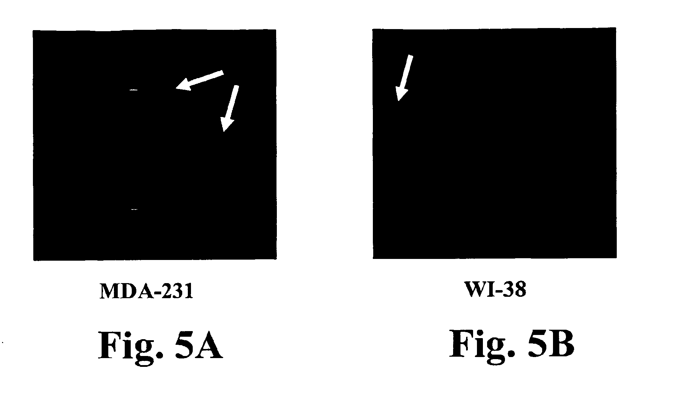

The results for MB-231 and WI-38 cells treated with 20 µM of gossypol for 24 hours as detected by the Hoechst Dye assay are shown in Figs. 5A and 5B, respectively. Treatment of MDA-MB-231 cancer cells with gossypol induces apoptosis in the cancer cells, but not in normal WI-38 fibroblast cells. Fig. 5A shows induction of apoptosis in the MDA-231 cells. Fig. 5B shows that gossypol treatment does not induce apoptosis in normal WI-38 fibroblast cells having low levels (*e.g.*, basal levels) of Bcl-2 and Bcl-X_{L} expression.

In other tests, gossypol was shown to induce apoptosis in T-47D breast cancer cells that have high levels of Bcl-X_{L} expression, but low levels of Bcl-2 expression. It was also found that gossypol induces apoptosis in other cancer cell lines with high Bcl-X_{L}, such as, human colon cancer cell line HT-29, but not in cancer cell lines with low Bcl-2 and low Bcl-X_{L} expression, such as, prostate cancer cell line DU-145.

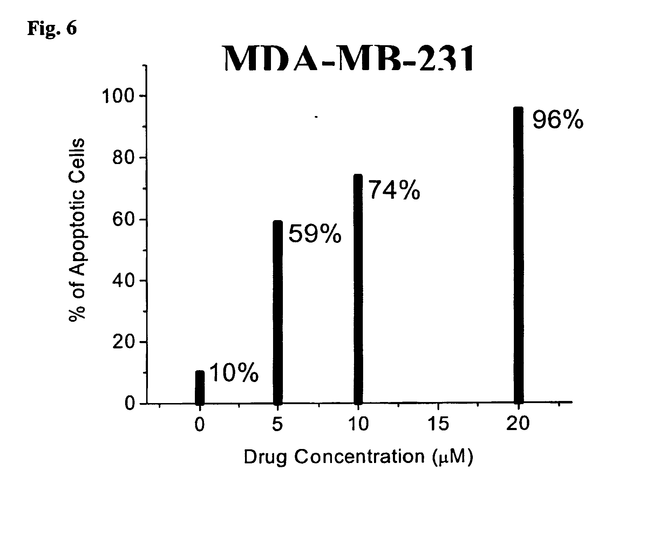

Further tests using an Annexin-V flow cytometry (FACS) assay were conducted to more quantitatively assess the potency of gossypol to induce apoptosis in MDA-MB-231 breast cancer cells. For example, Fig. 6 shows gossypol induced apoptosis in human MDA-MB-231 breast cancer cells treated with gossypol for 24 hours as detected using Annexin-V flow cytometry. It was observed that 5.0 µM of gossypol induced 59% of the cells undergoing apoptosis. Induction of apoptosis by gossypol is dose-dependent. At 10 and 20.0 µM gossypol induced 74% and 96% of cancer cells, respectively, to undergo apoptosis. MDA-231 overexpresses both Bcl-2 and Bcl-X_{L}.

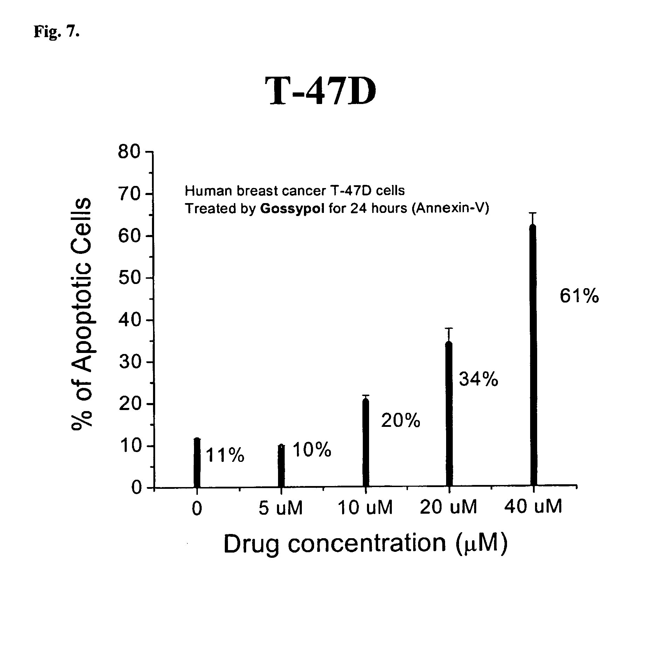

Since gossypol is a potent inhibitor of Bcl-X_{L}, the present invention contemplates that gossypol can induce apoptosis in cancer cells with high levels of Bcl-X_{L} but low level of Bcl-2. Indeed, gossypol induces dose-dependent apoptosis in human T-47D breast cancer cells, which as shown above, have high levels of Bcl-X_{L} but low levels of Bcl-2. Fig. 7 shows the dose dependent induction of apoptosis in human TD-47 cancer cells treated with gossypol for 24 hours as detected using Annexin-V flow cytometry.

These tests show that gossypol is a potent inhibitor of Bcl-X_{L} and induces cancer cells expressing high levels (*e.g.*, overexpression as compared to a basal expression rate for a normal example of the cell type) of Bcl-X_{L} to undergo apoptosis, but does not induce apoptosis in cells with normal levels of Bcl-2 and Bcl-X_{L} expression (*e.g.*, WI-38).

Some embodiments of the present invention, are related to (-)-gossypol for use in treating a subject having a condition characterized by the overexpression of Bcl-2 family proteins. The gossypol compounds contemplated for use in the present invention include, (-)-gossypol; and pharmaceutically acceptable salts of (-)-gossypol.

In particularly, preferred embodiments, the (-)-gossypol enantiomer (including derivatives, metabolites, Schiff's bases and pharmaceutically acceptable salts thereof) is administered to a patient.

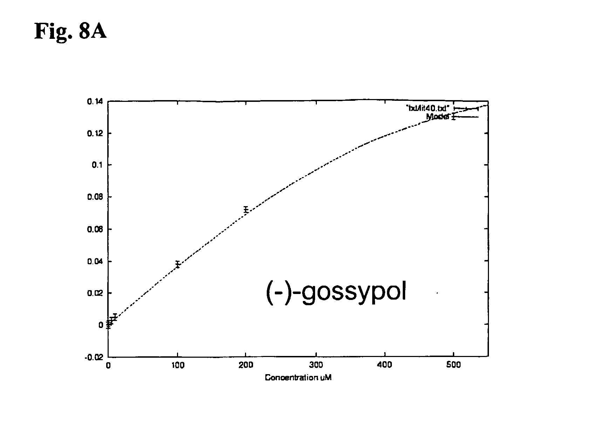

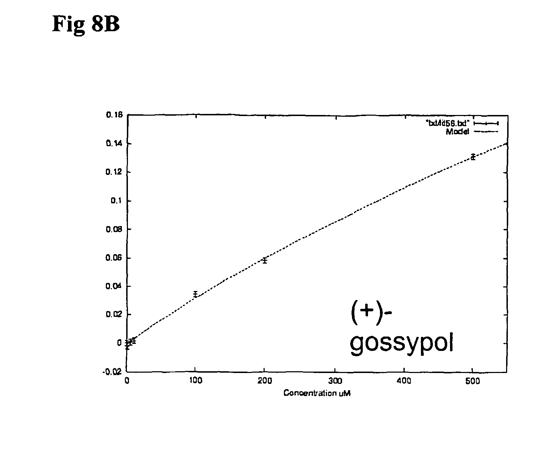

NMR analysis of the binding of (-)-gossypol and (+)-gossypol enantiomers conclusively show that both (-)-gossypol enantiomer and (+)-gossypol bind to Bcl-X_{L} (Figs. 8A and 8B).

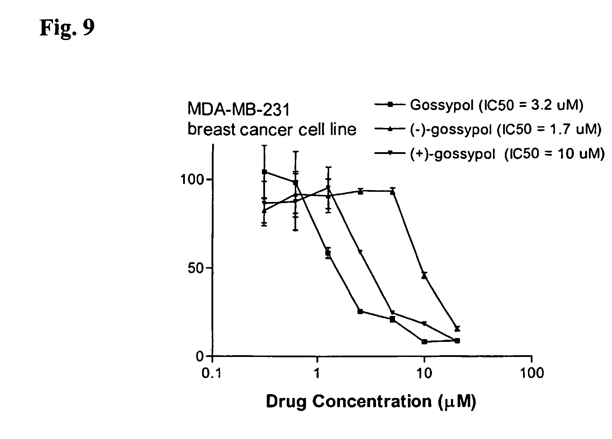

Fig. 9 shows data from growth inhibition experiments comparing (-)-gossypol and (+)-gossypol enantiomers and racemic gossypol in the MDA-MB-231 (2-LMP) breast cancer cell line (3000/200 µl/well using the MTT assay, 5 days; racemic gossypol IC₅₀ 3.2 µM; (-)-gossypol IC₅₀ 1.7 µM; and (+)-gossypol IC₅₀ 10µM).

Previous studies showed that the T_{1/2} of elimination of (+)-gossypol enantiomer in humans is 29 times that of (-)-gossypol. Therefore, the (+)-gossypol enantiomer is potentially more toxic than the (-)-gossypol enantiomer. Therefore, in some embodiments, the present invention contemplates that administration of (-)-gossypol enantiomer is potentially less toxic than the racemic form of gossypol or (+)-gossypol enantiomer.

In other embodiments, (-)-gossypol is (super-G) administered to patients with head-neck cancer characterized by the overexpression of a Bcl-2 family protein. Table 1, provided below, compares the inhibition of cell growth in a number of head-neck cancer cell lines by (-)-gossypol and cisplatin (a standard agent for the treatment head-neck cancer). Table 1 shows that (-)-gossypol potently inhibits the cancer cell growth in human head-neck cancer cell lines with elevated Bcl-X_{L} protein. These cancer cell lines are very resistant to cisplatin, a standard chemotherapy for the treatment of head-neck cancer.

**Table 1**

| | Super-G (IC₅₀, µM) | Bcl-X_{L} | Bcl-2 | cisplatin IC₅₀, µM) |
|---|---|---|---|---|
| UM-SCC-23 | 1.5 | +++ | - | 25 |
| UM-SCC-1 | 1.5 | +++ | - | 30 |
| UM-SCC-25 | 8 | + | - | 43 |

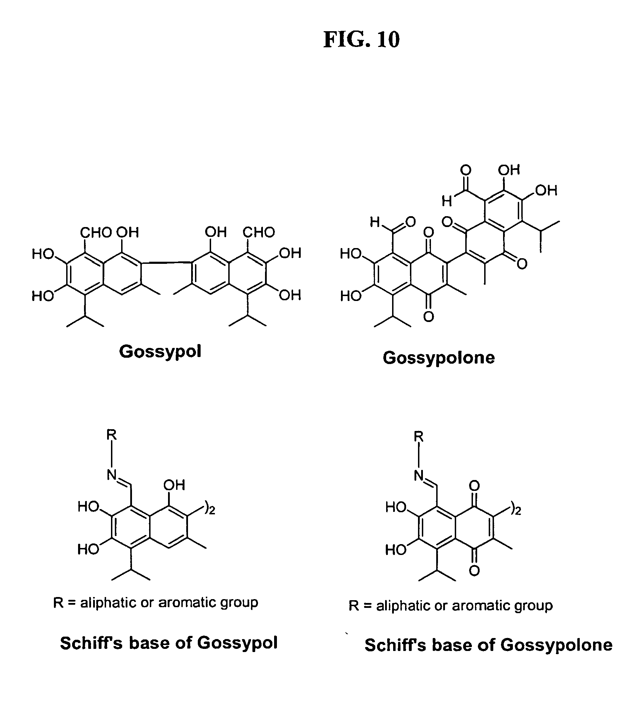

Fig. 10 provides the chemical structures of gossypol, gossypolone, and the Schiff's bases of gossypol and gossypolone.

### V. Proposed mechanism of gossypol activity

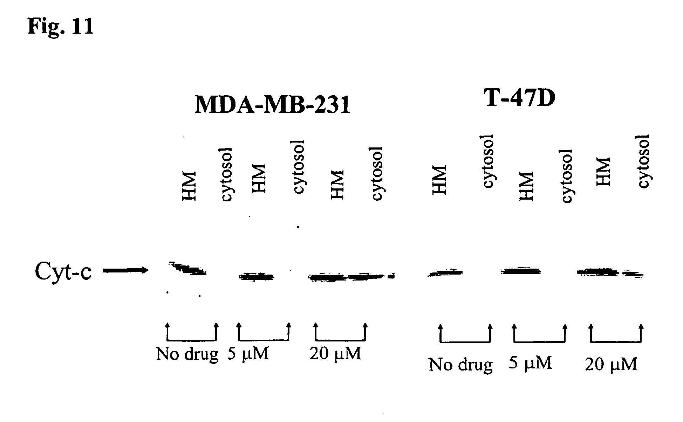

Although an understanding of the mechanism is not necessary to practice the present invention and the present invention is not so limited, it is contemplated that one of the key molecules in the Bcl-2/Bcl-X_{L} mediated apoptosis pathway is cytochrome-c (Cyt-c). Accordingly, it is further contemplated that one of the key functions of Bcl-2/Bcl-X_{L} is to heterodimerize with Bax, Bak, or Bad and block release of cyt-c from mitochondria. Thus, the ability of gossypol to induce Cyt-c release from mitochondria to the cytosol in cancer cells was tested. Breast cancer cell lines MDA-231 and T47D were treated with either 5 or 20 µM gossypol for 24 hours. Fig. 11, shows that Cyt-c was released from the mitochondria into the cytosol after treatment with 20 µM of gossypol in both the MDA-231 and T47D breast cancer cell lines (HM, Cyt-c found in the heavy membrane; Cytosol, Cyt-c found in the cytosol).

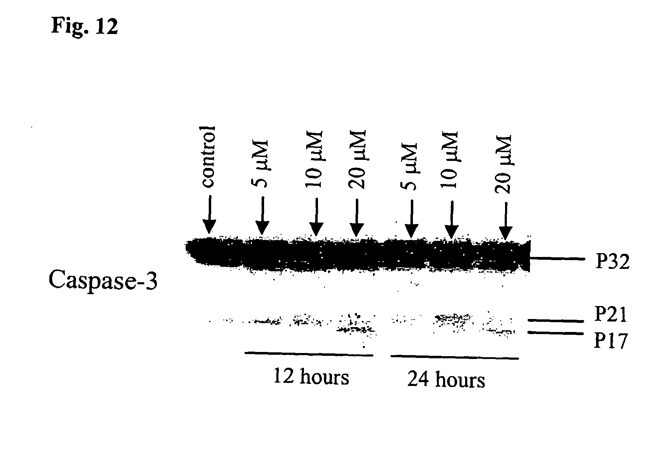

While not limited to any particular mechanism, it is also contemplated that Bcl-2 mediated apoptosis involves caspase (*e.g.*, caspase-3 and -9) activation once Cyt-c is released from the mitochondria. Therefore, tests were conducted to determine the whether gossypol activates caspase-3. The amount of caspase-3 cleavage in lysates of MDA-231 breast cancer cells was measured after 12 or 24 hours of treatment with gossypol. Fig. 12 shows that caspase-3 was cleaved after treatment with gossypol into 17 and 21 k_{D} fragments in a dose dependent manner. Similar results were obtained in T-47D human breast cancer cells and HT-29 colon cancer cells treated with gossypol, both of which have high levels of Bcl-X_{L} expression and relatively low levels of Bcl-2 after treatment with expression. In contrast, treatment of human DU-145 prostate cancer cells, having low Bcl-2 and Bcl-X_{L} expression, with 5, 10, or 20 µM of gossypol for 24 hours had no effect on caspase-3 activation. Therefore, activation of caspase-3 by gossypol is specific and correlative to expression levels of Bcl-X_{L} in cancer cells.

### VI. Activity of gossypol in MDA-231 xenograft mice alone and in combination with conventional anticancer agents

The potential of gossypol compound as an anticancer therapeutic agent was further evaluated in a human breast cancer MDA-MB-231 (subclone 2LMP) xenograft model in nude mice. A gossypol treatment regime was started at day 7 when mice tumors grew to 8-10 mm in diameter. Each treatment group had 5 mice bearing 10 human tumors (one tumor on each side). The control group had 5 mice to which no gossypol was administered. In the treated mice, gossypol was administered daily in two different oral doses, a 30 and a 90 mg/kg dose, for three weeks. It was found that at both 30 and 90 mg/kg daily doses, there is more than 70% inhibition of tumor growth by gossypol with more than a 95% confidence level at day 29. No weight loss or deaths were seen in the mice treated with gossypol. There did not appear to be any significant difference in the anticancer activity of gossypol in the 30 mg/kg and 90 mg/kg doses. These results suggest that at a 30 mg/kg daily dose of gossypol successfully inhibits tumor growth without supplementing the gossypol therapy with adjuvants or additional anticancer compounds or therapies.

Overexpression of Bcl-X_{L} appears to protects cancer cells from apoptosis induced by some conventional anticancer therapies (*e.g.*, docetaxel). Some embodiments of the present invention, therefore, provide administering an effective dose(s) of gossypol (and enantiomers, derivatives, and pharmaceutically acceptable salts thereof) in combination with at least one conventional anticancer therapy (*e.g.*, chemotherapeutic agents, such as, docetaxel, and/or radiation therapy).

In preferred embodiments, (-)-gossypol is administered in combination with one or more conventional anticancer therapies to treat diseases (*e.g.*, cancer) characterized by overexpression of Bcl-2 family proteins (*e.g.*, Bcl-2 and/or Bcl-X_{L}).

Although an understanding of the mechanism is not necessary to practice the present invention and the present invention is not so limited, it is contemplated that administration of (-)-gossypol in combination with a conventional anticancer therapy (*e.g.,* chemotherapeutics, such as, docetaxel) is contemplated to make cancer cells with high levels of expression of Bcl-2 family proteins (*e.g.*, Bcl-2 and/or Bcl-X_{L}) that are resistant to conventional anticancer therapies sensitive to treatment with gossypol and/or additional anticancer agents (*e.g.*, docetaxel). The present invention is, however, not limited to the administration of any particular combination of gossypol compounds and anticancer therapeutic agents, nor is the invention limited to any particular sequence or level of agents being administered.

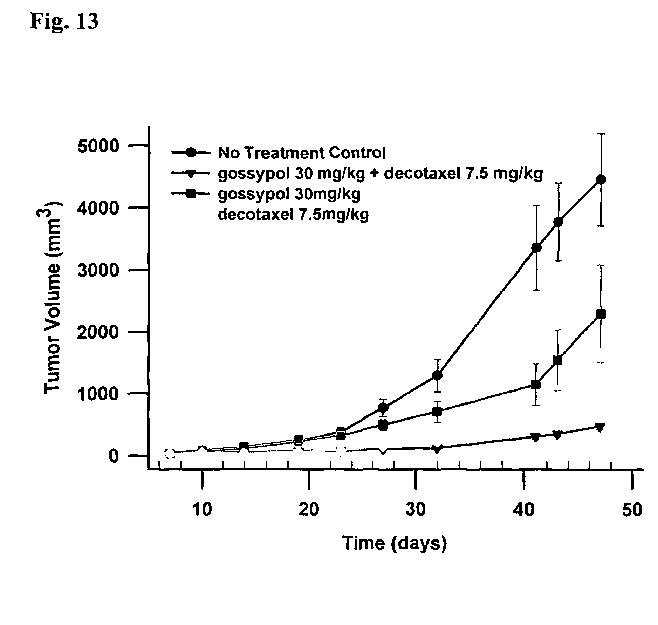

Since gossypol achieves significant inhibition of tumor growth at a daily dose of about 30 mg/kg, this dose level was selected for testing combinations of gossypol with other conventional anticancer therapeutics. A group of 10 mice received an orally administered daily dose of 30 mg/kg gossypol starting at day 7 and lasting for 4 weeks. Also starting at day 7, the same 10 mice were administered a weekly i.p. dose (7.5 mg/kg) of docetaxel for 3 weeks. The results of this experiment are shown in Fig. 13. In particular, Fig. 13 shows the inhibition of tumor growth by gossypol or docetaxel alone or in combination in human breast cancer xenograft MDA-MB-231 nude mice. Each experimental group had 10 animals. Fig. 13 shows that administration of gossypol alone (30 mg/kg daily), or docetaxel alone in a sub-optimal dose (7.5mg/kg weekly), significantly inhibited tumor growth in the test animals, however, test animals that received a combination therapy of gossypol and docetaxel showed even greater inhibition of tumor growth. Importantly, in the group, 3 out of 10 mice (6 tumors) treated with a combination gossypol and docetaxel showed complete tumor regression. Overall, there was more than 90% inhibition in tumor growth in the combination therapy group as compared to the group control. Statistical analyses were performed using the SAS (*See,* G. Verbeke and G. Molenberghs, Linear mixed models in practice: An SAS-orientated approach, Springer-Verlag, vol. 126 [1997]) program the results are provided in Table 2 shown below.

**Table 2**

| | Control | Gossypol | Docetaxel |
|---|---|---|---|
| Gossypol | 0.008^{*} | | |
| | (0.06^{‡}) | | |
| Docetaxel | 0.003 | | |
| | (0.01) | | |
| Gossypol + | 0.00001 | 0.005 | 0.01 |
| Docetaxel | (0.0000004) | (0.009) | (0.002) |

| | | | |
|---|---|---|---|
| ^{*} day 41 ^{‡} day 47 | | | |

The results show that the anticancer activity of gossypol and docetaxel is statistically significant as compared to controls at both day 41 and day 47 of the study. Furthermore, the anticancer activity of the combination of gossypol and docetaxel is significant as compared to the control (untreated) groups, and to the groups treated with gossypol or docetaxel alone. Taken together, these data indicate that gossypol has a significant anticancer activity alone, but in some embodiments achieves even greater activity when administered in combination with a conventional anticancer agent (*e.g.*, chemotherapeutic, such as, docetaxel).

Although an understanding of the mechanism is not necessary to practice the present invention and the present invention is not so limited, it is contemplated that the synergistic effects observed in some combinations of conventional anticancer agents and gossypol compounds is due to different mechanisms of action of the two compounds (*e.g.*, inhibition of the activity of Bcl-X_{L} by gossypol and targeting microtubule by decotaxel).

In other embodiments, again, although an understanding of the mechanism is not necessary to practice the present invention and the present invention is not so limited, it is contemplated that the synergistic effects observed in some combinations of conventional anticancer agents and gossypol compounds is due to similar mechanisms of action of the two compounds (*e.g.*, inhibition of the activity of Bcl-X_{L} by both gossypol and one or more additional anticancer agents).

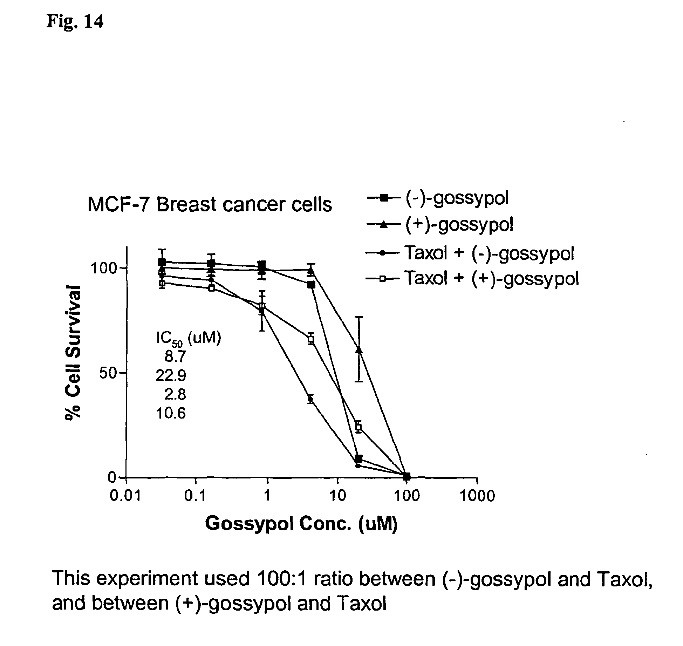

For example, in one embodiment (-)-gossypol enantiomer co-administered with the conventional anticancer therapeutic agent taxol provides a synergistic benefit. Fig. 14, shows the synergistic effects of co-administration of (-)-gossypol enantiomer and taxol. Briefly, this experiment used a 100:1 ratio of (-)-gossypol and (+)-gossypol enantiomers to taxol in a MCF-7 breast cancer cell line ((-)-gossypol IC₅₀ 8.71 µM; (+)-gossypol IC₅₀ 22.88 µM; taxol + (-)-gossypol IC₅₀ 2.755 µM; and taxol + (+)-gossypol IC₅₀ 10.57 µM).

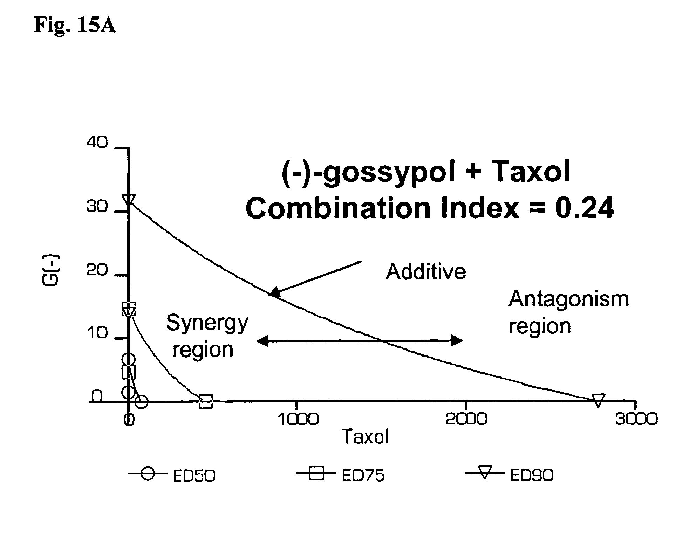

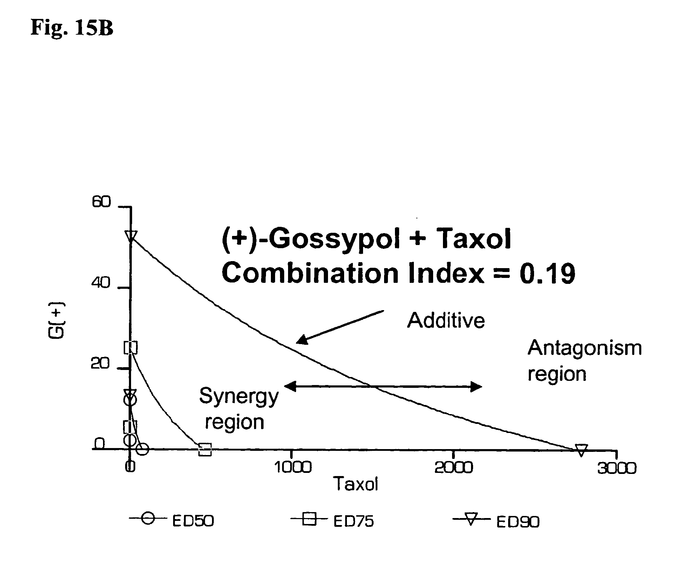

Similarly, Figs. 15A and 15B provide a combination index indicates that there is a very strong synergy between (-)-gossypol and taxol as well as between (+)-gossypol and taxol.

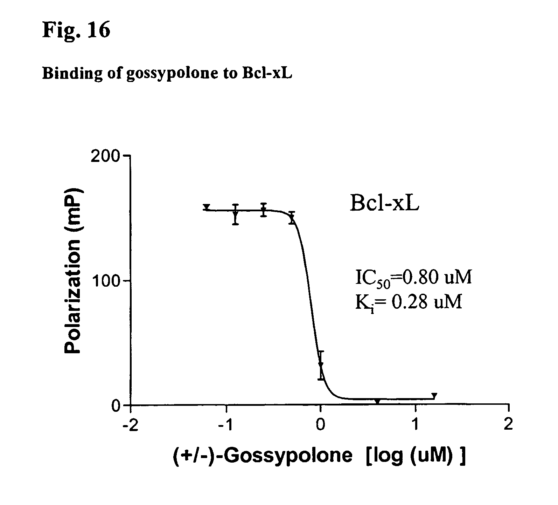

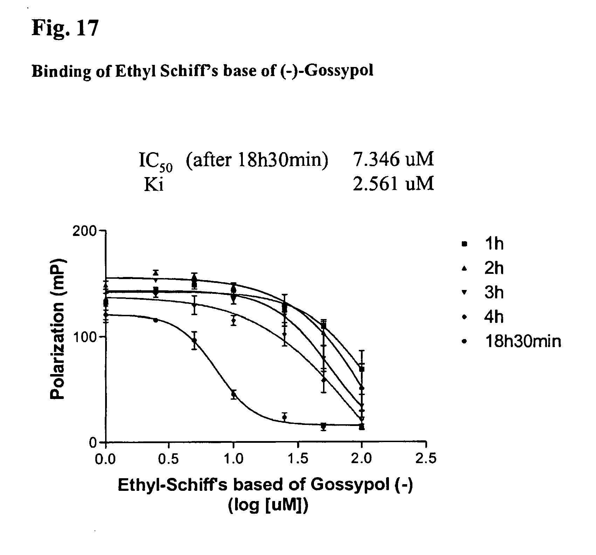

Fig. 16 shows the binding of gossypolone to Bcl-X_{L}. Fig. 17 shows Binding of Ethyl Schiff's base of (-)-gossypol.

### VII. Therapeutic Agents combined or co-administered with gossypol

A wide range of therapeutic agents find use with the present invention. Any therapeutic agent that can be co-administered with the gossypol compounds, or associated with gossypol compounds is suitable for use in the present invention.

Some embodiments of the resent invention provide administering to a subject an effective amount of (-)-gossypol (and enantiomers, derivatives, and pharmaceutically acceptable salts thereof) and at least one anticancer agent (*e.g.*, a conventional anticancer agent, such as, chemotherapeutic drugs, and/or radiation therapy). In some of these embodiments, the subject has disease characterized by intercellular overexpression of Bcl-2 family proteins (*e.g.*, Bcl-2 and/or Bcl-X_{L}).

Anticancer agent mechanisms suitable for use with the present invention include agents that induce apoptosis, agents that induce/cause nucleic acid damage, agents that inhibit nucleic acid synthesis, agents that affect microtubule formation, and agents that affect protein synthesis or stability.

Classes of anticancer agents suitable for use in compositions and methods of the present invention include, :1) alkaloids, including, microtubule inhibitors (*e.g*., Vincristine, Vinblastine, and Vindesine, *etc*.), microtubule stabilizers (*e.g.*, Paclitaxel [Taxol], and Docetaxel, *etc*.), and chromatin function inhibitors, including, topoisomerase inhibitors, such as, epipodophyllotoxins (*e.g.*, Etoposide [VP-16], and Teniposide [VM-26], *etc*.), and agents that target topoisomerase I (*e.g.*, Camptothecin and Isirinotecan [CPT-11], *etc.);* 2) covalent DNA-binding agents [alkylating agents], including, nitrogen mustards (*e.g.*, Mechlorethamine, Chlorambucil, Cyclophosphamide, Ifosphamide, and Busulfan [Myleran], *etc*.), nitrosoureas (*e.g.*, Carmustine, Lomustine, and Semustine, *etc*.), and other alkylating agents (*e.g.*, Dacarbazine, Hydroxymethylmelamine, Thiotepa, and Mitocycin, *etc*.); 3) noncovalent DNA-binding agents [antitumor antibiotics], including, nucleic acid inhibitors (*e.g.*, Dactinomycin [Actinomycin D], *etc*.), anthracyclines (*e.g.*, Daunorubicin [Daunomycin, and Cerubidine], Doxorubicin [Adriamycin], and Idarubicin [Idamycin], *etc*.), anthracenediones (*e.g.*, anthracycline analogues, such as, [Mitoxantrone], *etc*.), bleomycins (Blenoxane), *etc*., and plicamycin (Mithramycin), *etc*.; 4) antimetabolites, including, antifolates (*e.g.*, Methotrexate, Folex, and Mexate, *etc*.), purine antimetabolites (*e.g.*, 6-Mercaptopurine [6-MP, Purinethol], 6-Thioguanine [6-TG], Azathioprine, Acyclovir, Ganciclovir, Chlorodeoxyadenosine, 2-Chlorodeoxyadenosine [CdA], and 2'-Deoxycoformycin [Pentostatin], *etc*.), pyrimidine antagonists (*e.g.*, fluoropyrimidines [*e.g.*, 5-fluorouracil (Adrucil), 5-fluorodeoxyuridine (FdUrd) (Floxuridine)] *etc*.), and cytosine arabinosides (*e.g.*, Cytosar [ara-C] and Fludarabine, *etc.);* 5) enzymes, including, L-asparaginase, and hydroxyurea, *etc*.; 6) hormones, including, glucocorticoids, such as, antiestrogens (*e.g.*, Tamoxifen, *etc*.), nonsteroidal antiandrogens (*e.g.*, Flutamide, *etc*.), and aromatase inhibitors (*e.g.*, anastrozole [Arimidex], *etc.*); 7) platinum compounds (*e.g.,* Cisplatin and Carboplatin, *etc*.); 8) monoclonal antibodies conjugated with anticancer drugs, toxins, and/or radionuclides, *etc.;* 9) biological response modifiers (*e.g.*, interferons [*e.g.*, IFN-α, *etc*.] and interleukins [*e.g.*, IL-2, *etc*.], *etc.);* 10) adoptive immunotherapy; 11) hematopoietic growth factors; 12) agents that induce tumor cell differentiation (*e.g.*, all-trans-retinoic acid, *etc*.); 13) gene therapy techniques; 14) antisense therapy techniques; 15) tumor vaccines; 16) therapies directed against tumor metastases (*e.g.*, Batimistat, *etc*.); and 17) inhibitors of angiogenesis.

In preferred embodiments, the present invention provides administration of an effective amount of (-)-gossypol and at least one conventional anticancer agent that induces apoptosis to a subject. In some preferred embodiments, the subject has a disease characterized by overexpression of Bcl-2 family protein(s) (*e.g.*, Bcl-2 and/or Bcl-X_{L}). In yet other preferred embodiments, the present invention provides administration of an effective amount of gossypol and a taxane (*e.g.*, Docetaxel) to a subject having a disease characterized by the overexpression of Bcl-2 family protein(s) (*e.g.*, Bcl-2 and/or Bcl-X_{L}).

The taxanes (*e.g.*, Docetaxel) are an effective class of anticancer chemotherapeutic agents. (*See e.g.,* K.D. Miller and G.W. Sledge, Jr. Cancer Investigation, 17:121-136 [1999]). While the present invention is not intended to be limited to any particular mechanism, taxane-mediated cell death is though to proceed through intercellular microtubule stabilization and subsequent induction of the apoptotic pathway. (*See e.g.,* S. Haldar et al., Cancer Research, 57:229-233 [1997]). In many systems, Bcl-2 and Bcl-X_{L} function as a negative control on this pathway.

In some other embodiments, cisplatin and taxol are specifically contemplated for use with the gossypol compounds. Cisplatin and Taxol have a well-defined action of inducing apoptosis in tumor cells (*See e.g.,* Lanni et al., Proc. Natl. Acad. Sci., 94:9679 [1997]; Tortora et al., Cancer Research 57:5107 [1997]; and Zaffaroni et al., Brit. J. Cancer 77:1378 [1998]). However, treatment with these and other chemotherapeutic agents is difficult to accomplish without incurring significant toxicity. The agents currently in use are generally poorly water soluble, quite toxic, and given at doses that affect normal cells as wells as diseased cells. For example, paclitaxel (Taxol), one of the most promising anticancer compounds discovered, is poorly soluble in water. Paclitaxel has shown excellent antitumor activity in a wide variety of tumor models such as the B16 melanoma, L1210 leukemias, MX-1 mammary tumors, and CS-1 colon tumor xenografts. However, the poor aqueous solubility of paclitaxel presents a problem for human administration. Accordingly, currently used paclitaxel formulations require a cremaphor to solubilize the drug. The human clinical dose range is 200-500 mg. This dose is dissolved in a 1:1 solution of ethanol:cremaphor and diluted to one liter of fluid given intravenously. The cremaphor currently used is polyethoxylated castor oil. It is given by infusion by dissolving in the cremaphor mixture and diluting with large volumes of an aqueous vehicle. Direct administration (*e.g.*, subcutaneous) results in local toxicity and low levels of activity.
The present invention also provides the opportunity to monitor therapeutic success following delivery of cisplatin and/or Taxol to a subject. For example, measuring the ability of these drugs to induce apoptosis *in vitro* is reported to be a marker for *in vivo* efficacy (Gibb, Gynecologic Oncology, 65:13 [1997]). Therefore, in addition to the targeted delivery of either one or both of these drugs to provide effective anti-tumor therapy and reduce toxicity, the effectiveness of the therapeutic can be gauged by techniques of the present invention that monitor the induction of apoptosis. Importantly, both therapeutics are active against a wide-range of tumor types including, but not limited to, breast cancer and colon cancer (Akutsu et al., Eur. J. Cancer 31A:2341 [1995]).
Any pharmaceutical that is routinely used in a cancer therapy context finds use in the present invention. Conventional anticancer agents that are suitable for administration with the disclosed gossypol compounds include, but are mot limited to, adriamycin, 5-fluorouracil, etoposide, camptothecin, actinomycin-D, mitomycin C, or more preferably, cisplatin. These agent may be prepared and used as a combined therapeutic composition, or kit, by combining it with an immunotherapeutic agent, as described herein.
In related embodiments of the present invention, the therapeutic use of (-)-gossypol further comprise one or more agents that directly cross-link nucleic acids (*e.g.*, DNA) to facilitate DNA damage leading to a synergistic, antineoplastic agents of the present invention. Agents such as cisplatin, and other DNA alkylating agents may be used. Cisplatin has been widely used to treat cancer, with efficacious doses used in clinical applications of 20 mg/M² for 5 days every three weeks for a total of three courses. The compositions of the present invention may be delivered via any suitable method, including, but not limited to, injection intravenously, subcutaneously, intratumorally, intraperitoneally, or topically (*e.g.*, to mucosal surfaces).

Agents that damage DNA also include compounds that interfere with DNA replication, mitosis, and chromosomal segregation. Such chemotherapeutic compounds include, but are not limited to, adriamycin, also known as doxorubicin, etoposide, verapamil, podophyllotoxin, and the like. These compounds are widely used in clinical settings for the treatment of neoplasms, and are administered through bolus injections intravenously at doses ranging from 25-75 Mg/M² at 21 day intervals for adriamycin, to 35-50 Mg/M² for etoposide intravenously or double the intravenous dose orally.

Agents that disrupt the synthesis and fidelity of nucleic acid precursors and subunits also lead to DNA damage and find use as chemotherapeutic agents in the present invention. A number of nucleic acid precursors have been developed. Particularly useful are agents that have undergone extensive testing and are readily available. As such, agents such as 5-fluorouracil (5-FU) are preferentially used by neoplastic tissue, making this agent particularly useful for targeting to neoplastic cells. The doses delivered may range from 3 to 15 mg/kg/day, although other doses may vary considerably according to various factors including stage of disease, amenability of the cells to the therapy, amount of resistance to the agents and the like.

In preferred embodiments, the anticancer agents used in the present invention are those that are amenable co-administration with the disclosed gossypol compounds or are otherwise associated with the disclosed gossypol compounds such that they can be delivered into a subject, tissue, or cell without loss of fidelity of anticancer effect. For a more detailed description of cancer therapeutic agents such as a platinum complex, verapamil, podophyllotoxin, carboplatin, procarbazine, mechlorethamine, cyclophosphamide, camptothecin, ifosfamide, melphalan, chlorambucil, bisulfan, nitrosurea, adriamycin, dactinomycin, daunorubicin, doxorubicin, bleomycin, plicomycin, mitomycin, etoposide (VP16), tamoxifen, taxol, transplatinum, 5-fluorouracil, vincristin, vinblastin and methotrexate and other similar anti-cancer agents, those of skill in the art are referred to any number of instructive manuals including, but not limited to, the Physician's Desk reference and to Goodman and Gilman's "Pharmaceutical Basis of Therapeutics" ninth edition, Eds. Hardman et al., 1996.

In related embodiments, the drugs are attached to the gossypol compounds with photocleavable linkers. For example, several heterobifunctional, photocleavable linkers that find use with the present invention are described by Ottl *et al.* (Ottl et al., Bioconjugate Chem., 9:143 [1998]). These linkers can be either water or organic soluble. They contain an activated ester that can react with amines or alcohols and an epoxide that can react with a thiol group. In between the two groups is a 3,4-dimethoxy6-nitrophenyl photoisomerization group, which, when exposed to near-ultraviolet light (365 nm), releases the amine or alcohol in intact form. Thus, the therapeutic agent, when linked to the compositions of the present invention using such linkers, may be released in biologically active or activatable form through exposure of the target area to near-ultraviolet light.

In an exemplary embodiment, the alcohol group of taxol is reacted with the activated ester of the organic-soluble linker. This product in turn is reacted with the partially-thiolated surface of appropriate dendrimers (the primary amines of the dendrimers can be partially converted to thiol-containing groups by reaction with a sub-stoichiometric amount of 2-iminothiolano). In the case of cisplatin, the amino groups of the drug are reacted with the water-soluble form of the linker. If the amino groups are not reactive enough, a primary amino-containing active analog of cisplatin, such as Pt(II) sulfadiazine dichloride (Pasani et al., Inorg. Chim. Acta 80:99 [1983] and Abel *et al., **Eur. J. Cancer 9:4 [1973]) can be used. Thus conjugated, the drug is inactive and will not harm normal cells. When the conjugate is localized within tumor cells, it is exposed to laser light of the appropriate near-UV wavelength, causing the active drug to be released into the cell.

Similarly, in related embodiments of the present invention, the amino groups of cisplatin (or an analog thereof) is linked with a very hydrophobic photocleavable protecting group, such as the 2-nitrobenzyloxycarbonyl group (Pillai, V.N.R. Synthesis: 1-26 [1980]). When exposed to near-UV light (about 365 nm), the hydrophobic group is cleaved, leaving the intact drug. Since the drug itself is hydrophilic, it diffuses out of the dendrimer and into the tumor cell, where it initiates apoptosis.

An alternative to photocleavable linkers are enzyme cleavable linkers. A number of photocleavable linkers have been demonstrated as effective anti-tumor conjugates and can be prepared by attaching cancer therapeutics, such as doxorubicin, to water-soluble polymers with appropriate short peptide linkers (*See e.g.,* Vasey et al., Clin. Cancer Res., 5:83 [1999]). The linkers are stable outside of the cell, but are cleaved by thiolproteases once within the cell. In a preferred embodiment, the conjugate PK1 is used. As an alternative to the photocleavable linker strategy, enzyme-degradable linkers, such as Gly-Phe-Leu-Gly may be used.

For example, other conjugates that find use with the present invention include, but are not limited to, using conjugated boron dusters for BNCT (Capala et al., Bioconjugate Chem., 7:7 [1996]), the use of radioisotopes, and conjugation of toxins such as ricin.

Antimicrobial therapeutic agents may also be used as therapeutic agents in the present invention. Any agent that can kill, inhibit, or otherwise attenuate the function of microbial organisms may be used, as well as any agent contemplated to have such activities. Antimicrobial agents include, but are not limited to, natural and synthetic antibiotics, antibodies, inhibitory proteins, antisense nucleic acids, membrane disruptive agents and the like, used alone or in combination. Indeed, any type of antibiotic may be used including, but not limited to, anti-bacterial agents, anti-viral agents, anti-fungal agents, and the like.

In still further embodiments, another component of the present invention is that the gossypol compounds be associated with targeting agents (gossypol compound-targeting agent complex) that are able to specifically target a particular cell type (*e.g.,* tumor cell). Generally, the gossypol compound that is associated with a targeting agent, targets neoplastic cells through interaction of the targeting agent with a cell surface moiety and is taken into the cell through receptor mediated endocytosis.

Any moiety known to be located on the surface of target cells (*e.g.*, tumor cells) finds use with the present invention. For example, an antibody directed against such a moiety targets the compositions of the present invention to cell surfaces containing the moiety. Alternatively, the targeting moiety may be a ligand directed to a receptor present on the cell surface or vice versa. Similarly, vitamins also may be used to target the therapeutics of the present invention to a particular cell.

In related embodiments of the present invention, the targeting moiety may also function as an agent to identify a particular tumor characterized by expressing a receptor that the targeting agent (ligand) binds with, for example, tumor specific antigens including, but not limited to, carcinoembryonic antigen, prostate specific antigen, tyrosinase, ras, a sialyly lewis antigen, erb, MAGE-1, MAGE-3, BAGE, MN, gp100, gp75, p97, proteinase 3, a mucin, CD81, CID9, CD63; CD53, CD38, CO-029, CA125, GD2, GM2 and O-acetyl GD3, M-TAA, M-fetal or M-urinary find use with the present invention. Alternatively the targeting moiety may be a tumor suppressor, a cytokine, a chemokine, a tumor specific receptor ligand, a receptor, an inducer of apoptosis, or a differentiating agent.

Tumor suppressor proteins contemplated for targeting include, but are not limited to, p16, p21, p27, p53, p73, Rb, Wilms tumor (WT-1), DCC, neurofibromatosis type 1 (NF-1), von Hippel-Lindau (VHL) disease tumor suppressor, Maspin, Brush-1, BRCA-1, BRCA-2, the multiple tumor suppressor (MTS), gp95/p97 antigen of human melanoma, renal cell carcinoma-associated G250 antigen, KS 1/4 pan-carcinoma antigen, ovarian carcinoma antigen (CA125), prostate specific antigen, melanoma antigen gp75, CD9, CD63, CD53, CD37, R2, CD81, CO029, TI-1, L6 and SAS. Of course these are merely exemplary tumor suppressors and it is envisioned that the present invention may be used in conjunction with any other agent that is or becomes known to those of skill in the art as a tumor suppressor.

In preferred embodiments of the present invention, targeting is directed to factors expressed by an oncogene (*e.g., bcl-2* and/or *bcl-X_{L}*). These include, but are not limited to, tyrosine kinases, both membrane-associated and cytoplasmic forms, such as members of the Src family, serine/threonine kinases, such as Mos, growth factor and receptors, such as platelet derived growth factor (PDDG), SMALL GTPases (G proteins) including the *ras* family, cyclin-dependent protein kinases (cdk), members of the *myc* family members including *c-myc, N-myc,* and *L-myc* and *bcl-2* and family members.

Receptors and their related ligands that find use in the context of the present invention include, but are not limited to, the folate receptor, adrenergic receptor, growth hormone receptor, luteinizing hormone receptor, estrogen receptor, epidermal growth factor receptor, fibroblast growth factor receptor, and the like.

Hormones and their receptors that find use in the targeting aspect of the present invention include, but are not limited to, growth hormone, prolactin, placental lactogen, luteinizing hormone, foilicle-stimulating hormone, chorionic gonadotropin, thyroid-stimulating hormone, leptin, adrenocorticotropin (ACTH), angiotensin I, angiotensin II, α -endorphin, α melanocyte stimulating hormone (α -MSH), cholecystokinin, endothelin I, galanin, gastric inhibitory peptide (GIP), glucagon, insulin, amylin, lipotropins, GLP-1 (7-37) neurophysins, and somatostatin.

In addition, the present invention contemplates that vitamins (both fat soluble and non-fat soluble vitamins) used as targeting agents may be used to target cells that have receptors for, or otherwise take up these vitamins. Particularly preferred for this aspect are the fat soluble vitamins, such as vitamin D and its analogues, vitamin E, Vitamin A, and the like or water soluble vitamins such as Vitamin C, and the like.

In some embodiments of the present invention, any number of cancer cell targeting groups are associated with (-)-gossypol. Thus, (-)-gossypol associated with targeting groups are specific for targeting cancer cells (*i.e.*, much more likely to attach to cancer cells and not to healthy cells).

In preferred embodiments of the present invention, targeting groups are associated (*e.g.*, covalently or noncovalently bound) to (-)-gossypol with either short (*e.g.*, direct coupling), medium (*e.g.*, using small-molecule bifunctional linkers such as SPDP, sold by Pierce Chemical Company), or long (*e.g.*, PEG bifunctional linkers, sold by Shearwater Polymers) linkages.

In some embodiments, (-)-gossypol is associated with dendrimers (*e.g.*, PAMAM), or liposomes, or other carriers. Those skilled in the art will be able to readily design dendrimer gossypol compound molecules that take advantage of the multivalent structure of dendrimers.

In preferred embodiments of the present invention, the targeting agent is an antibody or antigen binding fragment of an antibody (*e.g*., Fab units). For example, a well-studied antigen found on the surface of many cancers (including breast HER2 tumors) is glycoprotein p185, which is exclusively expressed in malignant cells (Press et al., Oncogene 5:953 [1990]). Recombinant humanized anti-HER2 monoclonal antibodies (rhuMabHER2) have even been shown to inhibit the growth of HER2 overexpressing breast cancer cells, and are being evaluated (in conjunction with conventional chemotherapeutics) in phase III clinical trials for the treatment of advanced breast cancer (Pegrarn et al., Proc. Am. Soc. Clin. Oncol., 14:106 [1995]). Park *et al.* have attached Fab fragments of rhuMabHER2 to small unilamellar liposomes, which then can be loaded with the chemotherapeutic doxorubicin (dox) and targeted to HER2 overexpressing tumor xenografts (Park et al., Cancer Lett., 118:153 [1997] and Kirpotin et al., Biochem., 36:66 [1997]). These dox-loaded "immunoliposomes" showed increased cytotoxicity against tumors compared to corresponding non-targeted dox-loaded liposomes or free dox, and decreased systemic toxicity compared to free dox.

Antibodies can be generated to allow for the targeting of antigens or immunogens (*e.g.,* tumor, tissue or pathogen specific antigens) on various biological targets (*e.g.*, pathogens, tumor cells, normal tissue). Such antibodies include, but are not limited to polyclonal, monoclonal, chimeric, single chain, Fab fragments, and an Fab expression library.

In some preferred embodiments, the antibodies recognize tumor specific epitopes (*e.g.,* TAG-72 (Kjeldsen et al., Cancer Res. 48:2214-2220 [1988]; U.S. Pat. Nos. 5,892,020; 5,892,019; and 5,512,443); human carcinoma antigen (U.S. Pat. Nos. 5,693,763; 5,545,530; and 5,808,005); TP1 and TP3 antigens from osteocarcinoma cells (U.S. Pat. No. 5,855,866); Thomsen-Friedenreich (TF) antigen from adenocarcinoma cells (U.S. Pat. No. 5,110,911); "KC-4 antigen" from human prostrate adenocarcinoma (U.S. Pat. Nos. 4,708,930 and 4,743,543); a human colorectal cancer antigen (U.S. Pat. No. 4,921,789); CA125 antigen from cystadenocarcinoma (U.S. Pat. No. 4,921,790); DF3 antigen from human breast carcinoma (U.S. Pat. Nos. 4,963,484 and 5,053,489); a human breast tumor antigen (U.S. Pat. No. 4,939,240); p97 antigen of human melanoma (U.S. Pat. No. 4,918,164); carcinoma or orosomucoid-related antigen (CORA)(U.S. Pat. No. 4,914,021); a human pulmonary carcinoma antigen that reacts with human squamous cell lung carcinoma but not with human small cell lung carcinoma (U.S. Pat. No. 4,892,935); T and Tn haptens in glycoproteins of human breast carcinoma (Springer et al., Carbohydr. Res. 178:271-292 [1988]), MSA breast carcinoma glycoprotein termed (Tjandra et al., Br. J. Surg. 75:811-817 [1988]); MFGM breast carcinoma antigen (Ishida et al., Tumor Biol. 10:12-22 [1989]); DU-PAN-2 pancreatic carcinoma antigen (Lan et al., Cancer Res. 45:305-310 [1985]); CA125 ovarian carcinoma antigen (Hanisch et al., Carbohydr. Res. 178:29-47 [1988]); YH206 lung carcinoma antigen (Hinoda et al., Cancer J., 42:653-658 [1988]).

Various procedures known in the art are used for the production of polyclonal antibodies. For the production of antibody, various host animals can be immunized by injection with the peptide corresponding to the desired epitope including but not limited to rabbits, mice, rats, sheep, goats, *etc.* In a preferred embodiment, the peptide is conjugated to an immunogenic carrier (*e.g.*, diphtheria toxoid, bovine serum albumin (BSA), or keyhole limpet hemocyanin [KLH]). Various adjuvants are used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and *Corynebacterium parvum.*

For preparation of monoclonal antibodies, any technique that provides for the production of antibody molecules by continuous cell lines in culture may be used (*See e.g.,* Harlow and Lane, Antibodies: A Laboratory Manual, Cold Spring Harbor Laboratory Press, Cold Spring Harbor, NY). These include, but are not limited to, the hybridoma technique originally developed by Köhler and Milstein (Köhler and Milstein, Nature 256:495-497 [1975]), as well as the trioma technique, the human B-cell hybridoma technique (*See e.g.,* Kozbor et al., Immunol. Today 4:72 [1983]), and the EBV-hybridoma technique to produce human monoclonal antibodies (Cole et al., in Monoclonal Antibodies and Cancer Therapy, Alan R. Liss, Inc., pp. 77-96 [1985]).

In an additional embodiment of the invention, monoclonal antibodies can be produced in germ-free animals utilizing recent technology (*See e.g.,* PCT/US90/02545). According to the invention, human antibodies may be used and can be obtained by using human hybridomas (Cote et al., Proc. Natl. Acad. Sci. U.S.A.80:2026-2030 [1983]) or by transforming human B cells with EBV virus *in vitro* (Cole et al., in Monoclonal Antibodies and Cancer Therapy, Alan R. Liss, pp. 77-96 [1985]).

According to the invention, techniques described for the production of single chain antibodies (U.S. Patent 4,946,778) can be adapted to produce specific single chain antibodies. An additional embodiment of the invention utilizes the techniques described for the construction of Fab expression libraries (Huse et al., Science 246:1275-1281 [1989]) to allow rapid and easy identification of monoclonal Fab fragments with the desired specificity.

Antibody fragments that contain the idiotype (antigen binding region) of the antibody molecule can be generated by known techniques. For example, such fragments include but are not limited to: the F(ab')2 fragment that can be produced by pepsin digestion of the antibody molecule; the Fab' fragments that can be generated by reducing the disulfide bridges of the F(ab')2 fragment, and the Fab fragments that can be generated by treating the antibody molecule with papain and a reducing agent.

In the production of antibodies, screening for the desired antibody can be accomplished by techniques known in the art (*e.g.*, radioimmunoassay, ELISA (enzyme-linked immunosorbant assay), "sandwich" immunoassays, immunoradiometric assays, gel diffusion precipitin reactions, immunodiffusion assays, *in situ* immunoassays (using colloidal gold, enzyme or radioisotope labels, for example), Western Blots, precipitation reactions, agglutination assays (*e.g.*, gel agglutination assays, hemagglutination assays, *etc*.), complement fixation assays, immunofluorescence assays, protein A assays, and immunoelectrophoresis assays, *etc*.).

For breast cancer, the cell surface may be targeted with folic acid, EGF, FGF, and antibodies (or antibody fragments) to the tumor-associated antigens MUC1, cMet receptor and CD56 (NCAM).

A very flexible method to identify and select appropriate peptide targeting groups is the phage display technique (*See e.g.,* Cortese *et al.,* Curr. Opin. Biotechol., 6:73 [1995]), which can be conveniently carried out using commercially available kits. The phage display procedure produces a large and diverse combinatorial library of peptides attached to the surface of phage, which are screened against immobilized surface receptors for tight binding. After the tight-binding, viral constructs are isolated and sequenced to identify the peptide sequences. The cycle is repeated using the best peptides as starting points for the next peptide library. Eventually, suitably high-affinity peptides are identified and then screened for biocompatibility and target specificity. In this way, it is possible to produce peptides that can be conjugated to dendrimers, producing multivalent conjugates with high specificity and affinity for the target cell receptors (*e.g.*, tumor cell receptors) or other desired targets.

Related to the targeting approaches described above is the "pretargeting" approach (*See e.g.,* Goodwin and Meares, Cancer (suppl.) 80:2675 [1997]). An example of this strategy involves initial treatment of the patient with conjugates of tumor-specific monoclonal antibodies and streptavidin. Remaining soluble conjugate is removed from the bloodstream with an appropriate biotinylated clearing agent. When the tumor-localized conjugate is all that remains, a gossypol-linked, biotinylated agent is introduced, which in turn localizes at the tumor sites by the strong and specific biotin-streptavidin interaction.

In some embodiments of the present invention, the targeting agents (moities) are preferably nucleic acids (*e.g.*, RNA or DNA). In some embodiments, the nucleic acid targeting moities are designed to hybridize by base pairing to a particular nucleic acid (*e.g.*, chromosomal DNA, mRNA, or ribosomal RNA). In other embodiments, the nucleic acids bind a ligand or biological target. Nucleic acids that bind the following proteins have been identified: reverse transcriptase, Rev and Tat proteins of HIV (Tuerk et al., Gene, 137(1):33-9 [1993]); human nerve growth factor (Binkley et al., Nuc. Acids Res., 23(16):3198-205 [1995]); and vascular endothelial growth factor (Jellinek et al., Biochem., 83(34):10450-6 [1994]). Nucleic acids that bind ligands are preferably identified by the SELEX procedure (*See e.g.,* U.S. Pat. Nos. 5,475,096; 5,270,163; and 5,475,096; and in PCT publications WO 97/38134, WO 98/33941, and WO 99/07724.

### VIII. Pharmaceutical formulations, administration routes, and dosing considerations

To illustrate the delivery of therapeutic agents, the following discussion focuses mainly on the delivery of gossypol compounds and docetaxel for the treatment of cancer, however, the present invention is not intended to be limited to compositions and methods comprising the co-administration of docetaxel with gossypol compounds.

The present invention is defined in the claims and provides pharmaceutical compositions which may comprise at least one gossypol compound, and in preferred embodiments, at least one conventional anticancer agent, the gossypol compounds and anticancer agents may be administered in any sterile, biocompatible pharmaceutical carrier, including, but not limited to, saline, buffered saline, dextrose, and water. In some embodiments, the pharmaceutical compositions of the present invention may contain one agent (*e.g.*, a gossypol compound). In other embodiments, the pharmaceutical compositions may contain a mixture of at least two agents (*e.g.*, gossypol compounds and one or more conventional anticancer agents). In still further embodiments, the pharmaceutical compositions of the present invention contain at least two agents (*e.g.*, gossypol compounds and one or more conventional anticancer agents) that are administered to a patient under one or more of the following conditions: at different periodicities, different durations, different concentrations, different administration routes, *etc.*

The medical uses of the present invention find use in treating diseases or altering physiological states characterized by overexpression of Bcl-2 family proteins (*e.g.*, Bcl-2, Bcl-X_{L}, Mcl-1, A1/BFL-1, and BOO-DIVA, *etc*.). The invention provides methods for inducing apoptosis by administering antagonists of anti-apoptotic Bcl-2 family proteins, including, but not limited to, Bcl-2, Bcl-X_{L} Mcl-1, A1/BFL-1, and BOO-DIVA.

The present invention contemplates use of gossypol compounds and, in some embodiments, one or more conventional anticancer agents, in accordance with acceptable pharmaceutical delivery methods and preparation techniques. For example, gossypol compounds and suitable anticancer agents can be administered to a subject intravenously in a pharmaceutically acceptable carrier such as physiological saline. Standard methods for intracellular delivery of pharmaceutical agents can be used (*e.g.*, delivery via liposome). Such methods are well known to those of ordinary skill in the art.

In some embodiments, the formulations of the present invention are useful for parenteral administration, such as intravenous, subcutaneous, intramuscular, and intraperitoneal. Therapeutic co-administration of some contemplated anticancer agents (*e.g.*, therapeutic polypeptides) can also be accomplished using gene therapy as described above.

As is well known in the medical arts, dosages for any one patient depends upon many factors, including the patient's size, body surface area, age, the particular compound to be administered, sex, time and route of administration, general health, and interaction with other drugs being concurrently administered.

Accordingly, in some embodiments of the present invention, (-)-gossypol is administered to a patient alone, or in combination with one or more conventional anticancer agents (*e.g.*, nucleotide sequences, drugs, hormones, *etc*.) or in pharmaceutical compositions where it is mixed with excipient(s) or other pharmaceutically acceptable carriers. In one embodiment of the present invention, the pharmaceutically acceptable carrier is pharmaceutically inert.

Depending on the condition being treated, these pharmaceutical compositions may be formulated and administered systemically or locally. Techniques for formulation and administration may be found in the latest edition of "Remington's Pharmaceutical Sciences" (Mack Publishing Co, Easton Pa.). Suitable routes may, for example, include oral or transmucosal administration; as well as parenteral delivery, including intramuscular, subcutaneous, intramedullary, intrathecal, intraventricular, intravenous, intraperitoneal, or intranasal administration.

For injection, the pharmaceutical compositions of the invention may be formulated in aqueous solutions, preferably in physiologically compatible buffers such as Hanks' solution, Ringer's solution, or physiologically buffered saline. For tissue or cellular administration, penetrants appropriate to the particular barrier to be permeated are used in the formulation. Such penetrants are generally known in the art.

In other embodiments, the pharmaceutical compositions of the present invention can be formulated using pharmaceutically acceptable carriers well known in the art in dosages suitable for oral administration. Such carriers enable the pharmaceutical compositions to be formulated as tablets, pills, capsules, liquids, gels, syrups, slurries, suspensions and the like, for oral or nasal ingestion by a patient to be treated. In preferred embodiments, the gossypol compounds are administered orally to a patient orally.

Pharmaceutical compositions suitable for use in the present invention include compositions wherein the active ingredients are contained in an effective amount to achieve the intended purpose. For example, an effective amount of gossypol compounds may be that amount that induces apoptosis in a cell or tissue having elevated levels of a Bcl-2 family protein as compared to normal nonpathological examples of the particular cells or tissues. Determination of effective amounts is well within the capability of those skilled in the art, especially in light of the disclosure provided herein.

In addition to the active ingredients, preferred pharmaceutical compositions may contain suitable pharmaceutically acceptable carriers comprising excipients and auxiliaries that facilitate processing of the active compounds into preparations which can be used pharmaceutically. The preparations formulated for oral administration may be in the form of tablets, dragees, capsules, or solutions.

The pharmaceutical compositions of the present invention may be manufactured in a manner that is itself known (*e.g.*, by means of conventional mixing, dissolving, granulating, dragee-making, levigating, emulsifying, encapsulating, entrapping or lyophilizing processes).

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions.

Pharmaceutical preparations for oral use can be obtained by combining the active compounds (*e.g.*, gossypol compounds) with solid excipient, optionally grinding a resulting mixture, and processing the mixture of granules, after adding suitable auxiliaries, if desired, to obtain tablets or dragee cores. Suitable excipients are carbohydrate or protein fillers such as sugars, including lactose, sucrose, mannitol, or sorbitol; starch from corn, wheat, rice, potato, *etc*; cellulose such as methyl cellulose, hydroxypropylmethyl-cellulose, or sodium carboxymethylcellulose; and gums including arabic and tragacanth; and proteins such as gelatin and collagen. If desired, disintegrating or solubilizing agents may be added, such as the cross-linked polyvinyl pyrrolidone, agar, alginic acid or a salt thereof such as sodium alginate.

Ingestible formulations of the present compositions may further include any material approved by the United States Department of Agriculture for inclusion in foodstuffs and substances that are generally recognized as safe (GRAS), such as, food additives, flavorings, colorings, vitamins, minerals, and phytonutrients. The term phytonutrients as used herein, refers to organic compounds isolated from plants that have a biological effect, and includes, but is not limited to, compounds of the following classes: isoflavonoids, oligomeric proanthcyanidins, indol-3-carbinol, sulforaphone, fibrous ligands, plant phytosterols, ferulic acid, anthocyanocides, triterpenes, omega 3/6 fatty acids, polyacetylene, quinones, terpenes, cathechins, gallates, and quercitin.

Dragee cores are provided with suitable coatings such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, (*i.e.*, dosage).

Pharmaceutical preparations that can be used orally include push-fit capsules made of gelatin, as well as soft, sealed capsules made of gelatin and a coating such as glycerol or sorbitol. The push-fit capsules can contain the active ingredients mixed with a filler or binders such as lactose or starches, lubricants such as talc or magnesium stearate, and, optionally, stabilizers. In soft capsules, the active compounds may be dissolved or suspended in suitable liquids, such as fatty oils, liquid paraffin, or liquid polyethylene glycol with or without stabilizers.

Compositions comprising a compound of the invention formulated in a pharmaceutical acceptable carrier may be prepared, placed in an appropriate container, and labeled for treatment of an indicated condition. For gossypol compounds, conditions indicated on the label may include treatment of conditions related to faulty regulation of apoptosis, hyperproliferative diseases, cancers, acquired immune deficiency syndrome (AIDS), degenerative conditions, and vascular diseases. The pharmaceutical compositions may be provided as a salt and can be formed with many acids, including but not limited to hydrochloric, sulfuric, acetic, lactic, tartaric, malic, succinic, *etc.* Salts tend to be more soluble in aqueous or other protonic solvents that are the corresponding free base forms. In other cases, the preferred preparation may be a lyophilized powder in 1 mM-50 mM histidine, 0.1%-2% sucrose, 2%-7% mannitol at a pH range of 4.5 to 5.5 that is combined with buffer prior to use.

For any compound used in the method of the invention, the therapeutically effective dose can be estimated initially from cell culture assays. Then, preferably, dosage can be formulated in animal models (particularly murine models) to achieve a desirable circulating concentration range that induces apoptosis in cells with elevated levels of Bcl-2 family proteins. A therapeutically effective dose refers to that amount of gossypol compounds (and in some embodiments, one or more additional conventional anticancer agents) that ameliorate symptoms of the disease state (*e.g.*, unregulated cell proliferation diseases, including, but not limited to, cancer). Toxicity and therapeutic efficacy of such compounds can be determined by standard pharmaceutical procedures in cell cultures or experimental animals, *e.g.,* for determining the LD₅₀ (the dose lethal to 50% of the population) and the ED₅₀ (the dose therapeutically effective in 50% of the population). The dose ratio between toxic and therapeutic effects is the therapeutic index, and it can be expressed as the ratio LD₅₀/ED₅₀. Compounds that exhibit large therapeutic indices are preferred. The data obtained from cell culture assays and additional animal studies can be used in formulating a range of dosage, for example, mammalian use (*e.g.*, humans, *Equus caballus, Felis catus,* and *Canis familiaris, etc*.). The dosage of such compounds lies preferably, however the present invention is not limited to this range, within a range of circulating concentrations that include the ED₅₀ with little or no toxicity. The dosage varies within this range depending upon the dosage form employed, sensitivity of the patient, and the route of administration.

The exact dosage is chosen by the individual physician in view of the patient to be treated. Dosage and administration are adjusted to provide sufficient levels of the active moiety or to maintain the desired effect. Additional factors which may be taken into account include the severity of the disease state; age, weight, and gender of the patient; diet, time and frequency of administration, drug combination(s), reaction sensitivities, and tolerance/response to therapy. Long acting pharmaceutical compositions might be administered every 3 to 4 days, every week, or once every two weeks depending on half-life and clearance rate of the particular formulation. Other pharmaceutical compositions may be administered daily or several times a day.

Normal dosage amounts may vary from 0.1 to 100,000 micrograms, up to a total dose of about 1 g, depending upon the route of administration. Guidance as to particular dosages and methods of delivery is provided in the literature *(See,* U.S. Pat. Nos. 4,657,760; 5,206,344; or 5,225,212). Administration of some agents to a patient's bone marrow may necessitate delivery in a manner different from intravenous injections.

Preferred embodiments of the present invention provides pharmaceutical compositions and medical uses for administering an effective amount of (-)-gossypol to a patient to inhibit cell (*e.g.*, cancer cell) proliferation. In some other preferred embodiments, the present invention further provides pharmaceutical compositions and medical uses of coadministering an effective amount of at least one conventional anticancer agent in additional to (-)-gossypol to a patient, such that cell (*e.g.*, cancer cell) proliferation is inhibited.

In preferred embodiments, the subject has a disease characterized by the overexpression of a Bcl-2 family protein (*e.g.*, Bcl-2, Bcl-X_{L}, Mcl-1, A1/BFL-1, and BOO-DIVA, *etc*.). In some of embodiments, diseases characterized by overexpression of a Bcl-2 family protein include, but are not limited to, hyperproliferative diseases and cancers. In still further embodiments, the cancers suitable for treatment by the present compositions and methods, include, but are not limited to, breast cancer, prostate cancer, lymphoma, skin cancer, pancreatic cancer, colon cancer, melanoma, ovarian cancer, brain cancer, liver cancer, bladder cancer, non-small lung cancer, cervical carcinoma, myeloma, adrenal carcinoma, leukemia, neuroblastoma, and glioblastoma. However, the present invention is not intended to be limited to treating any particular type of cancer.

In some embodiments, diseases suspected of being characterized by having elevated levels of Bcl-2 family protein(s) suitable for treatment by the present invention are selected by obtaining a sample of interest (*e.g.*, cells, tissues, fluids, *etc.*) suspected of having high levels of Bcl-2 family proteins (*e.g.*, Bcl-2, Bcl-X_{L}, Mcl-1, A1/BFL-1, and BOO-DIVA, *etc*.), measuring the levels of Bcl-2 family proteins in the sample using one or more well established immunohistochemical techniques (*e.g.*, ELISA and Western blots, *etc.*), and comparing the levels of Bcl-2 family proteins in the sample with levels of Bcl-2 family proteins in relevant reference nonpathological samples. In other embodiments, diseases suspected of being characterized by having elevated levels of one or more Bcl-2 family proteins (*e.g.*, Bcl-2, Bcl-X_{L}, Mcl-1, A1/BFL-1, and BOO-DIVA, *etc*.) are selected by comparing levels of one or more markers (*e.g.*, polynucleotides, polypeptides, lipids, *etc.)* in a sample (*e.g.*, cells, tissues, fluids, *etc*.) that directly or indirectly indicate elevated levels of Bcl-2 family proteins in the sample as compared to levels of these markers relevant nonpathological samples. In still further embodiments, diseases suspected of being characterized by having elevated levels of Bcl-2 family proteins (*e.g.*, Bcl-2, Bcl-X_{L}, Mcl-1, A1/BFL-1, and BOO-DIVA, *etc*.) are selected from diseases that do not respond or that stop responding to treatment with one or more conventional anticancer therapies (*e.g.*, chemotherapy, radiation therapy, and/or surgical intervention).

The present invention is not intended to be limited to the administration routes chosen for delivering agents to a subject. Indeed, a number of suitable administration routes are contemplated the selection of which is within the skill of those in the art.

In some preferred embodiments, (-)-gossypol administered to a patient at a dosage range of about 1 to 200 mg/day, from about 5 to 50 mg/day, and most preferably from about 10 to 40 mg/day. In particularly preferred embodiments, (-)-gossypol is administered to a patient (*e.g.*, orally) in a tolerable daily dose (*e.g.,* 30 to 40 mg/day) shown to have some biologic activity (*e.g*., alterations in Rb and Cyclin D1 levels).

In still other preferred embodiments taxanes (*e.g.*, docetaxel) are administered to a patient in combination with (-)-gossypol. The classic docetaxel dosing schedule is 80-100 mg/m2 q 3 weeks. However, recent studies suggest that taxanes can be given safely, with perhaps higher dose intensity, on a q week schedule. *(See e.g.,* J. D. Hainsworth et al., J. Clin. Oncology, 16:2164-2168 [1998]; J. D. Hainsworth et al., J. Clin. Oncology, 19:3500-3505 [2001]; and C. Kouroussis et al., Cancer Chemo. Pharm., 46:488-492 [2000]). The patient toxicities associated with administering taxanes include, neutropenia, asthenia, alopecia, hypersensitivity reactions, skin toxicity, and edema. Preferred embodiments of the present invention provide weekly administrations of taxanes to reduce patient toxicities while preserving agent efficacy. In other embodiments, administration of taxanes (*e.g*., docetaxel) more frequently than once a week during a patient's course of treatment with the disclosed (-)-gossypol is expected to produce synergistic effects.

While the present invention is not limited to any particular mechanism, nor to any understanding of the action of the agents being administered, the following example provides a description of an exemplary testing procedure used determine potential drug interactions between (-)-gossypol and one or more anticancer agents that are candidates for co-administration with gossypol.

Docetaxel is extensively metabolized by CYP3A4, a specific cytochrome p450 enzyme. Pharmocokinetic data obtained for docetaxel indicates wide variance in its clearance between patients. Poor docetaxel clearance may result in an increase in the area-under-the-curve (AUC) and thus greater patient toxicity. Several investigations have reported that gossypol decreases cytochrome P-450 and mixed-function oxidases, although these results have been challenged, and no human studies have been performed which specifically address this issue. Thus, it is possible, that gossypol could inhibit CYP3A4 activity and lead to toxic docetaxel accumulation in some patients.

In one embodiment, a patient is administered a daily dose of gossypol for 1 week prior to receiving their first dose of docetaxel. The pharmocokinetic profile of docetaxel in the patient's system is evaluated after the patient receives her first dose of docetaxel. *(See,* R. Bruno et al., J. Clin. Oncol., 16:187-196 [1998]). Docetaxel dosing is started at a reduced dose of about 15 mg/m2/week. The dose of docetaxel will be gradually escalated to a maximally tolerated dose of about 35 mg/m2/week. Simultaneously, information will be collected on effects of gossypol administration on the phenotypic expression of CYP3A4.

The phenotypic expression of CYP3A4 can be measured easily and reproducibly using an erythromycin breath test (ERMBT). (*See, e.g.,* P. Watkins, Pharmacogenetics, 4:171-184 [1994]; and J. Hirth et al., Clin. Cancer Res., 6:1255-1258 [2000]). The ERMBT test has been shown to predict steady state trough blood levels of drugs that are CYP3A4 substrates. Consequently, some embodiments of the present invention are directed to the co-administration of (-)-gossypol and taxanes (*e.g.*, docetaxel) using the ERMBT to determine potential drug interactions between the gossypol and docetaxel. Those skilled in the art will appreciate that similar testing methodologies can be utilized to determine potential interactions between gossypol compounds and candidate compounds for co-administration.

In some embodiments, standard immunohistochemical techniques are employed on samples obtained from patients following, or during, treatments with the methods and compositions of the present invention to determine changes in the levels of Bcl-2 family proteins (*e.g*., Bcl-2, Bcl-X_{L}, and Bax, *etc*.). For example, in some embodiments, immunohistochemical techniques using antibodies to Bcl-2 (DAKO, Carpinteria, CA), Bcl-X_{L}, and/or Bax (Zymed, South San Francisco, CA) are used to determine the levels of these Bcl-2 proteins in patient samples. In preferred embodiments results from the immunohistochemical studies are interpreted using well-established criteria known to those in the art, any cytoplasmic or nuclear staining will be considered positive. The expression levels of Bcl-2, Bcl-X_{L}, and Bax will be determined by counting at least 1,000 neoplastic cells in each case and expressed as a percentage. Expression will be considered high when the percentage of positive cells is > 25% for Bcl-2, and Bcl-X_{L}, and >50% for Bax. *(See e.g.,* G. Rassidakis et al., Amer. J. Path., 159:527-535 (2001); and S. Shi et al., J. Histochem. Cytochem., 39:741-748 [1991]). In other embodiments, intermittent samples of whole blood will be obtained for fluorescence activated cell sorting (FACS) analysis for Bcl-2 and Bcl-X_{L} expression in peripheral blood lymphocytes (PBLs) and for immunomagnetic selection of circulating epithelial cells.

In preferred embodiments, the primary endpoint of dosing studies is obtained when the maximum tolerated dose of gossypol compound (at a particular daily dose, *e.g.,* 30 mg/day) administered in combination with a particular anticancer combination therapy candidate is established. In some embodiments, dose-limiting toxicity (DLT) is established when a given sample (*e.g.*, a cell, tissue, or fluid sample) shows >500 neutrophils or any other toxicity which is Grade 3 or 4 at any time while the patient is being studied.

In still some other embodiments, to evaluate dose escalation a minimum of 9 weeks of treatment is required for 2 patients started at each dose level. The maximum tolerated dose (MTD) is defined as the dose at which 33% of patients experience DLT. If the MTD is not reached by a particular dose then this dose level is defined as the MTD. In preferred embodiments, doses are allocated to patients according to the criteria described in the Continual Reassessment Method (J. O'Quigley et al., Biometrics 46:33-48 [1990]) called Time-to-Event CRM or (TITE-CRM). (K. Cheung and R. Chappell, Biometrics [in press]). Briefly, the TITE-CRM method assumes a model for the time to occurrence of toxic response as a function of dose, and allows information from all patients enrolled in the trial to be employed when allocating a new patient to a dose level. Because this method is very flexible in terms of the number of patients treated at each dose, subjects may be continuously recruited throughout a trial, without recruitment pauses, as long as patients are treated at a dose consistent with the safety profile.

In preferred embodiments, following treatment diseased cells and tissues are subjected to assays for cell viability, induction of apoptosis, such as, morphological changes, DNA integrity, mitochondria pathways, alterations of expression of Bcl-2 family proteins, and caspase activation as well as upstream and downstream effectors of caspases and caspase inhibitors. Those skilled in the art will be able to readily design and execute assays to test these and an number of other cellular and biochemical parameters in the treated cells and tissues.

### EXAMPLES

The following examples are provided to demonstrate and further illustrate certain preferred embodiments of the present invention and are not to be construed as limiting the scope thereof. The invention is defined in the claims.

### Example 1

### Homology modeling

The sequence of human Bcl-2 was obtained from Gene Bank (entry gi4557355) (SEQ ID NO:1). The NMR structure of Bcl-XL (pdb code: 1BXL from the protein databank), which has 45% amino acid sequence identity, 56% sequence similarity and 3% gaps with Bcl-2, was used as the template. The structure of Hcl-2 was built using the homology-modeling program MODELLER (version 4.0). (A. Sali et al., Structure, Function, and Genetics, 23:318-326 [1995]; and A. Sali, Curr. Opin. Biotech., 6:437-451 [1995]). Further refinement was done using the molecular dynamics program CHARMM (version 27b2). (B.R. Brooks, J. Comput. Chem., 4:187-217 [1983]). Hydrogen atoms were assigned to the modeled structure using the program QUANTA (Molecular Simulations Inc., San Diego, CA). The Bak BH3 peptide was placed into the Bcl-2 BH3 domain binding site in the same orientation as in the NMR structure of Bcl-XL in complex with the Bak BH3 peptide (1BXL in protein databank). (S. Michael et al., Science, 275:983-986 [1997]). The complex structure was solvated by inserting it into a 60 A diameter TIP3P water sphere and deleting solvent molecules that have heavy atoms at less then 2.5 A from any protein heavy atom.

The MD simulation was done using the all atom parameter set from the MSI CHARMM force field(ref 23) in QUANTA 98, a constant dielectric, ε = 1 and constant temperature, T = 300 K. The leap frog method with 1 fs time step was applied for numerical integration. Long-range electrostatic forces were treated with the force switch method with a switching range of 8-12 Å. *(See,* B.R. Brooks et al., J. Comput. Chem., 4:187-217 [1983]). Van der Waals forces were calculated with the shift method and a cutoff of 12 Å. The nonbond list was kept to 14 Å and updated heuristically. Solvent waters were kept from evaporating by using a spherical miscellaneous mean field potential as implemented in CHARMM. (B.R. Brooks, *supra).* The solvated protein was energy minimized using 100 cycles using the Steepest Descent method and additional 1000 cycles using the Adopted-Basis Newton Raphson method. This was followed by 3.0 ns MD simulation. The simulation was performed on an Origin2000 computer at the Advanced Biomedical Computing Center at the National Institutes of Health.

### Examples 2

### Expression and purification of the Bcl-X_{L} protein

Recombinant Bcl-X_{L} protein with an N-terminal His-tag was overexpressed from the pET15b expression vector (Novagen, Darmstadt, Germany). In this construct, the putative C-terminal membrane-anchoring region (residues 214-237) and a loop between helix 1 and helix 2 (residues 49-88) were removed to facilitate protein purification. This loop was previously shown to be dispensable for the anti-apoptotic activity of the protein. (*See,* S.W. Muchmore et al., Nature, 381:335-341 [1996]. The current construct of Bcl-X_{L} produces about 20 mg of the purified Bcl-X_{L} protein from 1L of cell culture.

The protein samples for NMR studies were uniformly labeled with ¹⁵N for screening and uniformly double labeled with ¹⁵N and ¹³C for structure characterization according to the methods described in M. Jansson et al., J. Biomol. NMR, 7:131-141 (1996), and M. L. Cai et al., J. Biomol. NMR, 11:97-102 (1998).

### Example 3

### Expression of Bcl-2 family proteins in human cancer cells

Several cancer cell lines (Table 3) that express various levels of Bcl-2 and/or Bcl-X_{L} proteins were selected in order to test the activity of gossypol to inhibit proliferation of human cancer cells.

**Table 3**

| | Cancer Cell Line | Bcl-2 expression | Bcl-X_{L} expression |
|---|---|---|---|
| Group I | T47 breast cancer MDA-453 breast cancer | ± - | +++ +++ |
| Group II | MDA-435 breast cancer | +++ | ± |
| Group III | MDA-231 breast cancer | +++ | +++ |
| Group IV | WI-38 normal SK-MEL-28 melanoma | - ± | - - |

Experiments using the well-characterized cancer cell lines shown in Table 3 allow for the testing of several important features of gossypol as a potential anticancer agent. First, the activity and selectivity of gossypol in binding assays with a number of cancer cell types that express various levels of Bcl-2 family proteins is expected to indicate the range of cancer types that are suitable candidates for treatment with gossypol. Second, testing gossypol in cancer cells with high Bcl-2 and high Bcl-X_{L} levels will indicate whether inhibition of one protein alone is sufficient for induction of apoptosis or whether simultaneous inhibition of both proteins will achieve a greater potency. Third, assays using a number of cancer cell types that express various levels of Bcl-2 family proteins will indicate whether gossypol displays selectivity in cancer cells with both low Bcl-2 and low Bcl-X_{L} expression.

### Example 4

### Fluorescence polarization based binding assays

The present invention uses an established sensitive and quantitative *in vitro* fluorescence polarization-based (FP) binding assays to determine the *in vitro* binding affinity of small molecule inhibitors (*e.g.*, gossypol compounds) to both Bcl-2 and Bcl-X_{L}, *(See e.g.,* J.L. Wang et al., Cancer Res., 60:1498-1502 [2000]; J.L. Wang et al., Proc. Natl. Acad. Sci. USA, 97:7124-7129 [2000]; A. Degterev et al., Nat. Cell Biolog., 3:173-182 [2001]; and I.J. Enyedy et al., J. Med. Chem., 44:4313-4324 [2001]). This assay monitors the displacement of a fluorescently labeled BH3 domain peptide from recombinant Bcl-2 or Bcl-X_{L} protein. Once a peptide binds to a Bcl-2 or Bcl-X_{L} protein, the fluorescence polarization is enhanced. When a small molecule inhibitor binds to Bcl-2 or Bcl-X_{L} and displaces the fluorescently labeled BH3 domain peptide, the fluorescence polarization is decreased. Determination of the binding affinities to both proteins is important to determine their selectivity. Although the structures of Bcl-2 and Bcl-X_{L} are very similar, there are certain differences between these two proteins. A small molecule inhibitor of Bcl-2/Bcl-X_{L} may display certain selectivity between these two proteins. Thus, the binding of gossypol to Bcl-2 and Bcl-X_{L} is further determined and confirmed using the NMR methods outlined herein.

Initial screening of Bcl-2 inhibitors (*e.g.*, gossypol, enantiomers, derivatives, and pharmaceutically acceptable salts thereof) is carried out at 200 µM. If significant inhibition is observed for an inhibitor (larger than 50%), its IC₅₀ value is determined. Five ml of the test compound in reaction buffer is added to wells containing tracer and Bcl-2 or Bcl-X_{L} protein provided at the same concentration. The final concentration of DMSO in all compounds should be less than 0.5%. The final reading is taken after a 10-min incubation at room temperature. For IC₅₀ determination of initial active compounds, 9 to 10 concentrations is used between 1 nM and 200 µM. The non-labeled Bak peptide is used as the positive control, while an inactive compound will be used as the negative control.

In one embodiment, Bcl-2 fluorescence polarization assays were carried out as follows. Fluorescein-labeled 16-mer peptide tracer Flu-Bak-BH3 (sequence GQVGRQLAIIGDDINR (SEQ ID NO:2) derived from Bak BH3 domain) were synthesized and labeled at the amino terminus. The 46-kDa recombinant soluble GST-fused Bcl-2 protein was purchased from Santa Cruz Biotechnology (Santa Cruz, CA). The reaction was carried out in a total volume of 20 µ per well containing 10 µl of 1X phosphoate-buffered saline, 5 µl of the GST-Bcl-2 protein, and 5 µl of peptide tracer. The reaction was incubated at room temperature for 20 min. The reading was taken at 485 nm and 535 nm using the Ultra Reader (Tecan U.S. Inc, Research Triangle Park, NC). A series of validation experiments were performed by analyzing the maximal and minimal signals obtained by the background, buffer, Bcl-2 protein, tracer and mixture of Bcl-2 protein and tracer. The K_{d} of binding between Bcl-2 protein and the 16-mer fluorescein-labeled peptide was determined by titration of Bcl-2 protein at a concentration range of 5.4 nM to 540 nM and fluorescent tracer concentration range of 0.145 nM to 1,450 nM. The optimal binding was obtained at a final concentration of 290 nM fluorescent tracer and 270 nM Bcl-2 protein. To verify the specificity, binding of the fluorescently labeled peptide was competed with nonlabeled 16-mer peptide. The data indicate that nonlabeled 16-mer peptide was able to abrogate binding of the labeled tracer, with an IC₅₀ of approximately 0.3 µM, a value similar to the value reported in the literature.

Initial screening of Gossypol and Gossypol analogues was carried out at 200 µM. Five micorliters of the test compound were added in reaction buffer to each of the wells containing tracer and Bcl-2 protein at the same concentration determined before. The final concentration of DMSO in all compounds was less than 1%. The final reading was taken after a 10 min incubation at room temperature. For IC₅₀ determination of active compounds, 6 to 7 point serial dilutions were made in triplicate starting at 200 µM.

Cells and reagents. Human breast cell lines (T47D, MDA-231, MDA-453), and human Leukemia cells HL-60 were obtained from the American Type Culture Collection (ATCC). All tumor cell lines were grown and maintained in RPMI 1640 medium containing 10% FCS, except MDA-MB-231, which used Dulbecco's modified Eagle's medium as basal medium. Cultures were maintained in a humidified incubator at 37 °C and 5% CO₂.

### Example 5

### Confirmation of gossypol binding to Bcl-X_{L} by NMR

The binding of gossypol to Bcl-X_{L} was determined using high resolution NMR techniques. Since, the NMR experiments were performed at pH 7.2, pulse field gradient (PFG) HSQC with water flip back was used to maximize the signal intensity (S. Grzesiek and A. Bax., J. Am. Chem. Soc., 115:12593-12594 [1993]; and G.S. Sheppard et al., Abstracts of Papers of the Amer. Chem. Soc., 213:81 [1997]) and to minimize the destruction from the water signal. HSQC spectra of Bcl-X_{L} were recorded prior to (free Bcl-X_{L}) and after the addition of the concentrated inhibitor solution. Then the two spectra were compared to identify the chemical shifts induced by the additions of the inhibitor. Data processing was conducted using nmrPipe, pipp and nmrDraw software *(See,* D.S. Garrett et al., J. Magn. Reson. Ser., B 95:214-220 [1991]; and F. Delaglio et al., J. Biomol. NMR, 6:277-293 [1995]). The shifted peaks were cross-referenced to the assignment table to reveal the residues affected by the presence of the inhibitors.

### Example 6

### Determination of the binding affinity of gossypol to Bcl-X_{L} by NMR

Similar HSQC experiments of Bcl-X_{L} will be performed to obtain the binding affinity of a small molecule inhibitor by titrating its concentration from nM to mM. Since the small molecule inhibitors bind to the Bcl-X_{L} protein in the fast exchange limit in the NMR time scale, some of the peak shifts occur in a stepwise manner towards the maximum shift positions with increasing amount of the inhibitors. The change in the chemical shift values versus the concentration of an inhibitor will be analyzed to obtain its K_{d} value. *(See e.g.,* P.E. Johnson et al., Biochemistry, 35:13895-13906 [1996]).

### Example 7

### Investigations into the mechanisms of apoptosis induced by gossypol

A series of biochemical assays is carried out to determine the mechanisms of action of the gossypol compounds as small molecule inhibitors of Bcl-2 family proteins (*e.g.*, Bcl-2 and/or Bcl-X_{L}). Although an understanding of the mechanism is not necessary to practice the present invention and the present invention is not so limited, it is contemplated that an understanding of the mechanisms by which gossypol compounds act on cells and tissue that overexpress Bcl-2 family proteins is advantageous to the present invention.

The expression and phosphorylation status of Bcl-2 proteins in cells treated with gossypol compounds at various doses and times is determined. In particular, the expression levels of Bcl-2, Bcl-X_{L}, Bcl-Xs, Bak, Bad, Bax, Bid is then be determined by specific antibodies using Western blot analysis. Phosphorylation status for proteins such as Bcl-2, Bcl-X_{L}, and Bad is determined with Western blot using specific antibodies recognize the phosphorylated proteins.

The effects of gossypol on cellular mitochondria will be examined by several methods. The two most important assays is cytochrome c release and pore formation assays. Cells is treated with inhibitors for 24-48 hours and cell fractionation is preformed as described in R.M. Kluck et al., Science, 275:1132-1136 (1997) with some modification. Briefly, cells are harvested and washed once with ice-cold PBS and resuspended in 1 ml ice-cold buffer C (10 mM Hepes-KOH at pH 7.4, 0.42 M NaCl, 2.5% (v/v) glycerol, 1.5 mM MgCl₂, 0.5 mM sodium EDTA, 0.5 mM EGTA, 1 mM dithiothreitol) and a protease inhibitor mix (PIM). The cell suspension is homogenized on ice by passage through 15 times through a 22 gauge needle. The homogenates are centrifuged twice at 750g for 10 min at 4°C to remove nuclei. The post-nuclear supernatant fractions are centrifuged at 10,000g for 15 min at 4°C, and the resulting mitochondria-enriched pellets are resuspended in 100 ml buffer C + PIM (cold). The post-mitochondrial supernatant is centrifuged at 10,000g for 1 hrs at 4°C to remove membrane contaminants and the resulting supernatant (soluble portion) is used for cytosolic cytochrome-c release detection, the pellet is the mitochondrial membrane (heavy membrane proteins) portion. Soluble fraction proteins and an equivalent amount of heavy membrane proteins is subjected to SDS-PAGE and analyzed by Western blotting with antibodies against cytochorme c (Becton Dickinson, Franklin Lakes, NJ), voltage-dependent anion channel (VDAC) (Calbiochem, La Jolla, CA), Smac, and AIF (apoptosis inducing factor) (Santa Cruz Bitoechnology, Santa Cruz, CA). This determines apoptosis signaling following treatment of the cells (*e.g.*, cancer cells) with gossypol compound through three of the mitochondrial pathways: cyto-c release, Smac release, and AIF release.

The activation of caspases is determined by collecting and washing treated cells with PBS and suspended in 25 mM HEPES (pH 7.5), 5 mM MgCl₂, 5 mM EDTA, 5 mM dithiothione, 2 mM phenylmethanesulfonyl fluoride, 10 g/mL pepstatin A, and 10 g/mL leupeptin after treatment. The treated cells are then lysed. The cell lysates are clarified by centrifugation at 12000xg for 20 min at 4°C. Caspase-1, -2, -3, -6, -8, and -9 activity in the supernatant is determined by the fluorogenic CaspACE Assay System (Promega Corp., Madison, WI). Briefly, 50 mg of total protein, as determined by bicinchoninic acid assay (Promega Corp.), is incubated with 50 mM substrate Ac-YVAD-AMC, Ac-VDVAD-AMC, Ac-DEVD-AMC, Ac-VEID-AMC, Ac-IETD-AMC, or Ac-LEHD-AMC at 30 C for 1 hour. The release of methylcoumaryl-7-amine (AMC) is measured by excitation at 360 nm and emission at 460 nm using a fluorescence spectrophotometer (Hitachi F-4500) according to G. Denecker et al., Cell Mol. Life Sci., 58:356-370 (2001); and V.M. Kolenko et al., Apoptosis, 5:17-20 [2000]).

The effects of caspase inhibitors (*e.g.*, Z-VAD-FMK, non-specific, Z-DEVD-FMK, caspases-3, -6, -7, -8, and -10, and Z-LEHD-FMK, caspase-9) on the activity of the gossypol compounds using standard caspase inhibitor assays. All of these caspase inhibitors are commercially available (Enzyme System Products, Livermore, CA).

### Example 8

### Cell survival assay

Cells are seeded in 24 or 96-well-plates and gossypol compounds are prepared at 2-10 fold dilutions in medium. Inhibition of cell viability is determined by treatment of cells with the gossypol compounds for 24 hrs and determined by the trypan-blue assay. Inhibition of cell growth is determined by treatment of cells with the inhibitors for 3-6 days and determined by the MTT assay.

The MTT assay is a fast, accurate, and reliable methodology for obtaining cell viability measurements. The MTT assay was first developed by Mosmann (Mosmann, J. Immunol. Meth., 65:55 [1983]). It is a simple colorimetric assay numerous laboratories have utilized for obtaining toxicity results *(See e.g.,* Kuhlmann et al., Arch. Toxicol., 72:536 [1998]). Briefly, the mitochondria produce ATP to provide sufficient energy for the cell. In order to do this, the mitochondria metabolize pyruvate to produce acetyl CoA. Within the mitochondria, acetyl CoA reacts with various enzymes in the tricarboxylic acid cycle resulting in subsequent production of ATP. One of the enzymes particularly useful in the MTT assay is succinate dehydrogenase. MTT (3-(4,5-dimethylthiazol-2-yi)-2 diphenyl tetrazolium bromide) is a yellow substrate that is cleaved by succinate dehydrogenase forming a purple formazan product. The alteration in pigment identifies changes in mitochondria function. Nonviable cells are unable to produce formazan, and therefore, the amount produced directly correlates to the quantity of viable cells. Absorbance at 540 nm is utilized to measure the amount of formazan product.

The results of the *in vitro* tests can be compared to *in vivo* toxicity tests in order to extrapolate to live animal conditions. Typically, acute toxicity from a single dose of the substance is assessed. In some embodiments, animals are monitored over 14 days for any signs of toxicity (increased temperature, breathing difficulty, death, etc). Traditionally, the standard of acute toxicity is the median lethal dose (LD50), which is the predicted dose at which half of the treated population would be killed. The determination of this dose occurs by exposing test animals to a geometric series of doses under controlled conditions. Other tests include subacute toxicity testing, which measures the animal's response to repeated doses of the gossypol compound (or one or more conventional anticancer agents) for no longer than 14 days. Subchronic toxicity testing involves testing of a repeated dose for 90 days. Chronic toxicity testing is similar to subchronic testing but may last for over a 90 day period. *In vivo* testing can also be conducted to determine toxicity with respect to certain tissues. For example, in some embodiments of the present invention, tumor toxicity (*i.e.*, effect of the compositions of the present invention on the survival of tumor tissue) is determined (*e.g.*, by detecting changes in the size and/or growth of tumor tissues).

Following the treatment of cells with inhibitors, qualitative assessments of cell morphology are made to determine features related to apoptosis such as cellular swelling, nuclear swelling, membrane blebbing, vacuolization and apoptotic body formation.

In some embodiments, DNA level detection of apoptosis is made by detecting DNA fragmentation using the TUNEL assay. When cells are undergoing apoptosis, apoptotic endonucleases not only affect cellular DNA by producing the classical DNA ladder but also generate free 3' -OH groups at the ends of these DNA fragments. These groups are end-labeled by TdT-FragFLTM DNA Fragmentation Kit (Oncogene, Boston, MA) allowing for the detection of apoptotic cells using molecular biology-based, end-labeling, histochemical, or cytochemical techniques. The rational of this assay is that terminal deoxynucleotidyl transferase (TdT) binds to exposed 3' -OH ends of DNA fragments generated in response to apoptotic signals, and catalyses the addition of biotin-labeled and unlabeled deoxynucleotides. Biotinylated nucleotides are detected using a strepavidin-horseradish peroxidase (HRP) conjugate. Diaminobenzidine reacts with the labeled sample to generate an insoluble colored substrate at the site of DNA fragmentation. *(See e.g.,* L. Lagneaux et al., Br. J. Haematol., 112:344-352 (2001); and K. Kitamura, Leukemia, 14:1743-1750 [2000]).

In other embodiments, nuclei level detection methods (*e.g.*, fluorescent dye hoechst 33258 and propidium iodide staining) were used to quantify signs of apoptosis in the treated cells. Morphological changes in the nuclear chromatin of cells undergoing apoptosis were detected by staining with 2.5 µg/ml bisbenzimide Hoechst 33258 fluorochrome (Calbiochem, La Jolla, CA) followed by examination on a fluorescence microscope. In some experiments, cells were double-stained with propidium iodide (PI, 2.5 µg/ml) and Hoechst 33258 (2.5 µg/ml) to distinguish apoptotic cells from necrotic cells. Intact blue nuclei, condensed/fragmented blue nuclei, condensed/fragmented pink nuclei, and intact pink nuclei were considered viable, early apoptotic, late apoptotic, and necrotic cells, respectively. *(See e.g.,* B. R. Gastman et al. Cancer Res., 60:6811-6817 (2000); and N., Hail Jr., and R. Lotan, R. Cancer Epidemiol. Biomarkers Prev., 9:1293-1301 [2000]).

In still further embodiments, the present invention uses cell level detection of apoptotic events by flow cytometry. Cells undergone apoptotic events were detected by flow cytometry using a FACSCAN (Becton Dickinson, Franklin Lakes, NJ) with 488-nm laser line and analyzed using Cell Quest software (Becton Dickinson). Phosphatidylserine exposed on the outside of the cells (one of the major characteristics of apoptosis) is determined by TACSTM Annexin V-FITC Kit (Trevigen, Gaithersburg, MD). Annexin V-FITC fluorescence is detected in FL-1, and propidium iodide is detected in FL-2. (Hail Jr., and R. Lotan, R. Cancer Epidemiol. Biomarkers Prev., 9:1293-1301 [2000]).

### Example 9

### Colony-formation in soft-agarose

The soft-agar colony formation assay is used to directly measure the transforming ability of cancer cells. This assay is known to correlate well with *in vivo* tumorigenicity.

Previously, it was reported that when Bcl-2 antisense G3139 alone inhibited cell proliferation, thus, combination treatments with chemotherapeutic drugs are contemplated to yield better effects. Experiments are conducted using combination treatments to assess the apoptotic effects or inhibition of cell proliferation of Bcl-2 inhibitors in combination with known chemotherapeutic agents. In preferred embodiments, these results are used to select synergistic agent combinations.

### Example 10

### In vivo antitumor activity studies

Preliminary *in vivo* studies showed that gossypol is a potent Bcl-X_{L} inhibitor, and that it exhibits significant anti-tumor activity alone and in combination with additional conventional anticancer agents (*e.g.*, Docetaxel). Preferred embodiments of the present invention provide thorough *in vivo* anti-tumor efficacy and selectivity studies using human cancer xenograft models.

In order to design optimal dose schedules for gossypol therapies, studies first utilize human breast cancer cell lines MDA-231 (clone 2LMP), and then additional tumor xenograft models such as MDA-435 (LCC6), and T47D. MDA-231 expresses high levels of both Bcl-2 and Bcl-X_{L}; MDA-435 expresses high levels Bcl-2 but low levels of Bcl-X_{L}; T47D expresses low levels of Bcl-2 but high levels of Bcl-X_{L}. Among all the 7 human breast cancer cell lines examined, no cell line has both low Bcl-2 and low Bcl-X_{L}. Human prostate DU-145 mice xenograft model, however, expresses low levels of both Bcl-2 and Bcl-X_{L} and is thus as a negative control in some embodiments to test the specificity of gossypol and gossypol derivatives.

In the MDA-231 model, a series of comprehensive doses and schedule investigations is performed to determine: 1) the minimal active dose, defined as inhibition of tumor growth by 50% as compared to control with a statistical confidence level of 95%; 2) the optimal schedule of administration in inhibition of tumor growth while not causing toxicity defined as weight loss of more than 25%; 3) the effect of gossypol on large tumors (more than 2,000 mm³); 4) how long can gossypol be administered to mice in the control group without causing morbidity or mortality (*e*.*g*., weight loss).

After identifying the optimal dose and schedule, testing combination therapies with at least one additional conventional chemotherapeutic agents is conducted, including, but not limited to, 1) Doxorubicin (4 mg/kg); 2) 5-FU (10 mg/kg); 3)VP-16/Etoposide (40 mg/kg); 4) Cyclophosphamide (100 mg/kg); 5) Cisplatin (10 mg/kg). As a positive control, Taxotere will be used at a dose of 7.5 mg/kg. Control group mice receive either no treatment or vehicle alone. To achieve statistic significance, a minimum of 10 mice per group is used in the combination regimes.

For all tests, mice are randomized and then injected into the fat pad with 1-5 x10⁶ cells prepared in serum-free medium. The animals are measured and weighed twice each week during the treatment period, followed by twice a week measurements for an additional 4-6 weeks. A gross visual necropsy of each animal will be performed at death or terminal sacrifice.

The rate apoptosis in tissues is determined using the TUNEL method (terminal deoxyribonucleotidyl transferase [TdT]-mediated dUTP-digoxigenin nick end labeling) to detect apoptosis in tissues and cells. This method is extremely sensitive and allows for determination of very few apoptotic cells *in situ.* (*See,* Y. Gavrieli et al., J. Cell Biol., 119:493-501 [1992]; and M. Dowsett et al., Cytometry, 32:291-300 [1998]). Paraffin-embedded tissues are sectioned, and slides are incubated in labeling buffer for 5 minutes and then in a humid chamber with TdT, dNTP mix, and Mg⁺⁺ in labeling buffer. Strepavidin-Horseradish Peroxidase is applied onto each sample for 10 min., washed and counter stained with methyl green or hematoxylin. Apoptosis rate is calculated by counting and dividing the number of apoptotic cells by the total number of cells *See*n per light microscopy field at 40x magnification, and are expressed by percent.

### Example 11

### Drug interactions between gossypol and Docetaxel

The effect of gossypol alone on the erythromycin breath test (ERMBT), a phenotypic test for CYP3A4 metabolism, is evaluated by comparing baseline ERMBT level at baseline and after 1-week pretreatment with gossypol. (*See, e*.*g*., P. Watkins, Pharmacogenetics, 4:171-184 [1994]; and J. Hirth et al., Clin. Cancer Res., 6:1255-1258 [2000]). The ERMBT produces a measurement of the percentage of ¹⁴C exhaled per hour, and is usually approximated using the Normal distribution. (*See*, D. Wagner, Clin. Pharm. Therap., 64:129-130 [1998]). The mean levels of ¹⁴C exhaled/h will be compared using a standard (alpha = 0.05), two-sided, paired t test. Using estimates from previous work (J. Hirth et al., Clin. Cancer Res., 6:1255-1258 (2000) for the baseline mean ¹⁴C exhaled/h (n = 21, mean = 2.41), its standard deviation (SD =1.08), correlation of ERMBT measurements over time (r = 0.81), and assuming constant variance at each measurement, the current study with data from 30 subjects has 96% power to detect a 20% decrease (2.41 to 1.93), and 81% power to detect a 15% decrease (2.41 to 2.05) in mean ERMBT levels. The mean, standard deviation, and range of ERMBT values will be tabulated and reported, along with the significance of the paired t test.

### Example 12

### Pharmacokinetic description of docetaxel in patient samples

Blood is drawn for pharmacokinetic description of docetaxel when treated in combination with gossypol using an optimal sampling strategy. (*See,* P. Baille et al., Clin. Cancer Res., 3:1535-1538 [1997]). Blood is drawn directly before the end of infusion (EOI) of docetaxel, at 0.25, 0.75, 3.00, 6.50, and at 24 hrs following EOI. All samples are assayed at the same time. A Bayesian criterion is used to calculate the docetaxel plasma concentration area under the curve (AUC) based upon measured drug levels and population pharmacokinetic parameters previously estimated (See, R. Bruno et al., J. Clin. Oncol., 16:187-196 [1998]) for docetaxel alone, using NONMEM software. (S.L. Beal and L.B. Shener, Nonlinear Mixed Effects Model Users Guides (San Francisco, CA, NONMEM Project Group, University of California at San Francisco), 1999). Clearance (CL) is directly estimated by fitting the nonlinear mixed effects model. (R. Bruno et al., J. Clin. Oncol., 16:187-196 [1998]). The AUC is calculated as a function of dose and clearance, defined AUC = dose/CL. ERMBT values from baseline and following 1-week gossypol pretreatment is plotted separately against Clearance. Ordinary least squares regression is used to defined the relationship between Docetaxel CL and the ERMBT or the natural logarithm of ERMBT. The slopes estimated are displayed along with previously published values for single treatment docetaxel CL (J. Hirth et al., Clin. Cancer Res., 6:1255-1258 (2000) in order to describe any differences in docetaxel CL when administered in combination with gossypol versus single agent administration.

### Example 13

### Expression of Bcl-2 family protein in patient samples

The expression of Bcl-2 and Bel-X_{L} in tissue samples collected in paraffin blocks is assayed using standard immunohistochemical (IHC) assay methods. IHC results are dichotomized and tabulated to report the percentage of patients expressing these markers. Results are also tabulated against anti-tumor response. A Chi-square statistic or Fisher's exact test is performed to assess the relationship between expression and anti-tumor response, depending on the size of the expected cell counts of the resulting table.

### Example 14

### Peripheral blood lymphocytes studies

Blood samples collected from study participants before study treatment, on day 8 (following 1 week gossypol pretreatment), and at week 9 (after completion of one cycle of gossypol plus docetaxel) are used for exploratory studies involving peripheral blood lymphocytes (PBL) and circulating epithelial cells (CEC). Using fluorescent activated cell sorting techniques (FACS), PBLs are characterized for their expression of Bcl-2 and Bel-X_{L} before and after gossypol administration. The mean number of PBLs with expression and its standard deviation will be reported at each time point. Using an immunomagnetic separation method previously described (See, T. Walker et al., Proc. Amer. Soc. Clin. Oncology, 19:54b [2001]) the feasibility of finding CECs is determined for each patient. The mean number of CECs per 10 ml of blood collected and its standard deviation across patients at each time point will be reported.

## Claims

1. Use of (-)-gossypol in the manufacture of a medicament for the treatment of cancer in a subject, wherein a therapeutically effective amount of said (-)-gossypol is co-administered to said subject with an anticancer agent selected from the group consisting of docetaxel and paclitaxel.

2. The use of claim 1, wherein said cancer is selected from the group consisting of breast cancer, prostate cancer, lymphoma, skin cancer, pancreatic cancer, colon cancer, melanoma, ovarian cancer, brain cancer, head and neck cancer, liver cancer, bladder cancer, non-small lung cancer, cervical carcinoma, leukemia, neuroblastoma, glioblastoma, adrenal carcinoma, and myeloma.

3. The use of claim 2, wherein said cancer is selected from the group consisting of breast cancer and prostate cancer.

4. The use of claim 3, wherein said cancer is prostate cancer.

5. The use of claim 4, wherein said anticancer agent is docetaxel.

6. The use of claim 1, wherein said subject is human.

7. The use of any one of claims 1-6, wherein the co-administration of (-)-gossypol and docetaxel or paclitaxel produces synergistic anticancer activity in said subject.

8. (-)-Gossypol for use in a method for treating cancer in a subject, wherein a therapeutically effective amount of said (-)-gossypol is co-administered to said subject with an anticancer agent selected from the group consisting of docetaxel and paclitaxel.

9. The (-)-gossypol of claim 8, wherein said cancer is selected from the group consisting of breast cancer, prostate cancer, lymphoma, skin cancer, pancreatic cancer, colon cancer, melanoma, ovarian cancer, brain cancer, head and neck cancer, liver cancer, bladder cancer, non-small lung cancer, cervical carcinoma, leukemia, neuroblastoma, glioblastoma, adrenal carcinoma, and myeloma.

10. The (-)-gossypol of claim 9, wherein said cancer is selected from the group consisting of breast cancer and prostate cancer.

11. The (-)-gossypol of claim 10, wherein said cancer is prostate cancer.

12. The (-)-gossypol of claim 11, wherein said anticancer agent is docetaxel.

13. The (-)-gossypol of claim 8, wherein said subject is human.

14. The (-)-gossypol of any one of claims 8-13, wherein the co-administration of (-)-gossypol and docetaxel or paclitaxel produces synergistic anticancer activity in said subject.

## Patentansprüche

1. Verwendung von (-)-Gossypol zur Herstellung eines Medikaments zur Behandlung von Krebs in einem Subjekt, worin eine therapeutisch wirksame Menge des (-)-Gossypols mit einem Antikrebs-Agens ausgewählt aus der Gruppe bestehend aus Docetaxel und Paclitaxel an ein Subjekt zusammen verabreicht wird.

2. Verwendung nach Anspruch 1, worin der Krebs ausgewählt ist aus der Gruppe bestehend aus Brustkrebs, Prostatakrebs, Lymphom, Hautkrebs, Pankreaskrebs, Kolonkrebs, Melanom, Ovarienkrebs, Gehirnkrebs, Kopf- und Nackenkrebs, Leberkrebs, Blasenkrebs, nicht-kleiner ("non-small") Lungenkrebs, Zervixkarzinom, Leukämie, Neuroblastom, Glioblastom, Nierenkrebs und Myelom.

3. Verwendung nach Anspruch 2, worin der Krebs ausgewählt ist aus der Gruppe bestehend aus Brustkrebs und Prostatakrebs.

4. Verwendung nach Anspruch 3, worin der Krebs Prostatakrebs ist.

5. Verwendung nach Anspruch 4, worin das Antikrebs-Agens Docetaxel ist.

6. Verwendung nach Anspruch 1, worin das Subjekt ein Mensch ist.

7. Verwendung nach jedem der Ansprüche 1-6, worin die gemeinsame Verabreichung von (-)-Gossypol und Docetaxel oder Paclitaxel eine synergistische Antikrebs-Aktivität in dem Subjekt produziert.

8. (-)-Gossypol zur Verwendung in einem Verfahren zur Behandlung von Krebs in einem Subjekt, worin eine therapeutisch wirksame Menge des (-)-Gossypols mit einem Antikrebs-Agens ausgewählt aus der Gruppe bestehend aus Docetaxel und Paclitaxel an ein Subjekt zusammen verabreicht wird.

9. (-)-Gossypol nach Anspruch 8, worin der Krebs ausgewählt ist aus der Gruppe bestehend aus Brustkrebs, Prostatakrebs, Lymphom, Hautkrebs, Pankreaskrebs, Kolonkrebs, Melanom, Ovarienkrebs, Gehirnkrebs, Kopf- und Nackenkrebs, Leberkrebs, Blasenkrebs, nicht-kleiner ("non-small") Lungenkrebs, Zervixkarzinom, Leukämie, Neuroblastom, Glioblastom, Nierenkrebs und Myelom.

10. (-)-Gossypol nach Anspruch 9, worin der Krebs ausgewählt ist aus der Gruppe bestehend aus Brustkrebs und Prostatakrebs.

11. (-)-Gossypol nach Anspruch 10, worin der Krebs Prostatakrebs ist.

12. (-)-Gossypol nach Anspruch 11, worin das Antikrebs-Agens Docetaxel ist.

13. (-)-Gossypol nach Anspruch 8, worin das Subjekt ein Mensch ist.

14. (-)-Gossypol nach jedem der Ansprüche 8-13, worin die gemeinsame Verabreichung von (-)-Gossypol und Docetaxel oder Paclitaxel eine synergistische Antikrebs-Aktivität in dem Subjekt produziert.

## Revendications

1. Utilisation de (-)-gossypol dans la fabrication d'un médicament destiné au traitement du cancer chez un sujet, dans laquelle une quantité thérapeutiquement efficace dudit (-)-gossypol est co-administrée audit sujet avec un agent anticancéreux sélectionné dans le groupe consistant le docétaxel et le paclitaxel.

2. Utilisation selon la revendication 1, dans laquelle ledit cancer est sélectionné dans le groupe consistant en un cancer du sein, un cancer de la prostate, un lymphome, un cancer de la peau, un cancer du pancréas, un cancer du côlon, un mélanome, un cancer des ovaires, un cancer du cerveau, un cancer de la tête et du cou, un cancer du foie, un cancer de la vessie, un cancer des poumons non à petites cellules, un carcinome du col de l'utérus, une leucémie, un neuroblastome, un glioblastome, un carcinome surrénalien, et un myélome.

3. Utilisation selon la revendication 2, dans laquelle ledit cancer est sélectionné dans le groupe consistant en un cancer du sein et un cancer de la prostate.

4. Utilisation selon la revendication 3, dans laquelle ledit cancer est un cancer de la prostate.

5. Utilisation selon la revendication 4, dans laquelle ledit agent anticancéreux est le docétaxel.

6. Utilisation selon la revendication 1, dans laquelle ledit sujet est humain.

7. Utilisation selon l'une quelconque des revendications 1 à 6, dans laquelle la co-administration de (-)-gossypol et de docétaxel ou de paclitaxel produit une activité anticancéreuse synergistique chez ledit sujet.

8. (-)-Gossypol destiné à être utilisé dans un procédé de traitement du cancer chez un sujet, où une quantité thérapeutiquement efficace dudit (-)-gossypol est co-administrée audit sujet avec un agent anticancéreux sélectionné dans le groupe consistant en le docétaxel et le paclitaxel.

9. (-)-Gossypol selon la revendication 8, où ledit cancer est sélectionné dans le groupe consistant en un cancer du sein, un cancer de la prostate, un lymphome, un cancer de la peau, un cancer du pancréas, un cancer du côlon, un mélanome, un cancer des ovaires, un cancer du cerveau, un cancer de la tête et du cou, un cancer du foie, un cancer de la vessie, un cancer des poumons non à petites cellules, un carcinome du col de l'utérus, une leucémie, un neuroblastome, un glioblastome, un carcinome surrénalien, et un myélome.

10. (-)-Gossypol selon la revendication 9, où ledit cancer est sélectionné dans le groupe consistant en un cancer du sein et un cancer de la prostate.

11. (-)-Gossypol selon la revendication 10, où ledit cancer est un cancer de la prostate.

12. (-)-Gossypol selon la revendication 11, où ledit agent anticancéreux est le docétaxel.

13. (-)-Gossypol selon la revendication 8, où ledit sujet est humain.

14. (-)-Gossypol selon l'une quelconque des revendications 8 à 13, où la co-administration de (-)-gossypol et de docétaxel ou de paclitaxel produit une activité anticancéreuse synergistique chez ledit sujet.
